# EUROPEAN PATENT APPLICATION

(11) **EP 4 170 041 A1**
(43) Date of publication of application: **26.04.2023**
(21) Application number: 21825674.1
(22) Date of filing: 16.06.2021
(51) Int. Cl.: C12Q 1/26, C12M 1/34, C12N 9/02, C12N 15/53

(54) **METHOD FOR QUANTIFYING ETHANOLAMINE PHOSPHATE, OXIDE REDUCTASE FOR QUANTIFICATION, QUANTIFICATION COMPOSITION, QUANTIFICATION KIT, SENSOR CHIP, AND SENSOR**

(30) Priority: 17.06.2020 JP 2020104881
(71) Applicant: Kikkoman Corporation, Noda-shi, Chiba 278-8601 (JP)
(72) Inventor: HIRAGUCHI Haruka, Noda-shi, Chiba 278-8601 (JP); ICHIYANAGI Atsushi, Noda-shi, Chiba 278-8601 (JP)
(74) Representative: dompatent von Kreisler Selting Werner - Partnerschaft von Patent- und Rechtsanwälten mbB
(86) International application number: PCT/JP2021/022845
(87) International publication number: WO 2021/256503

(57) **Abstract**

A quantitation method of ethanolamine phosphate capable of measuring trace amounts of ethanolamine phosphate, or oxidoreductase used for the quantitation method thereof, a quantitation composition, a quantitation kit, a sensor chip, or a sensor is provided. According to an embodiment of the present invention, a quantitation method of ethanolamine phosphate including allowing phosphatase to act on a sample to convert ethanolamine phosphate contained in the sample into ethanolamine, and allowing oxidoreductase to act on the ethanolamine is provided.

## Description

### TECHNICAL FIELD

The present invention relates to a quantitation method of ethanol amine phosphate, an oxidoreductase for quantitation, a quantitation composition, a quantitation kit, a sensor chip, and a sensor.

### BACKGROUND ART

Ethanolamine phosphate (EAP) contained in human blood is known to be a biomarker for diagnosing depression (Patent Literature 1). In addition, Non-Patent Literature 1 describes that it is possible to diagnose depression with a concentration of EAP of 1.5 µM or less in plasma as a reference value.

However, a complicated measurement method such as capillary electrophoresis time-of-flight mass spectrometry has been used for the measurement method of EAP. Patent Literature 2 describes a method for measuring a concentration of EAP using an EAP phospholyase and an acetaldehyde dehydrogenase as a simpler measurement method.

### CITATION LIST

### PATENT LITERATURE

Patent Literature 1: International patent publication No. WO2011/019072
Patent Literature 2: International patent publication No. WO2013/069645

### NON-PATENT LITERATURE

Non-Patent Literature 1: Kawamura N, "Plasma metabolome analysis of patients with major depressive disorder." Psychiatry Clin Neurosci. 2018 May;72(5):349-361

### SUMMARY OF INVENTION

### TECHNICAL PROBLEM

As described above, in order to diagnose depression, although a concentration of EAP of 1.5 µM or less in plasma is set as a reference value, quantitation of trace amounts of EAP has not been realized by the measurement method of EAP concentration using an EAP phospholyase in Patent Literature 2. Therefore, there is a need for a new measurement method capable of measuring trace amounts of EAP.

An embodiment of the present invention provides a quantitation method of EAP capable of measuring trace amounts of EAP. Alternatively, an embodiment of the present invention provides oxidoreductase for quantitation used in the quantitation method. Alternatively, an embodiment of the present invention provides a quantitation composition used in the quantitation method. Alternatively, an embodiment of the present invention provides a quantitation kit used in the quantitation method. Alternatively, an embodiment of the present invention provides a sensor chip used in the quantitation method. Alternatively, an embodiment of the present invention provides a sensor used in the quantitation method.

### SOLUTION TO PROBLEM

According to an embodiment of the present invention, there is provided a quantitation method of EAP including allowing phosphatase to act on a sample to convert EAP contained in the sample into ethanolamine (EA), and allowing oxidoreductase to act on the EA.

In the quantitation method of EAP, the sample includes at least one kind of amine other than the EAP, the oxidoreductase is an oxidoreductase acting on at least the amine, and the oxidoreductase may be allowed to act on the at least one kind of amine before allowing phosphatase to act.

In the quantitation method of EAP, a mediator is reduced by allowing the oxidoreductase to act on the EA, and a concentration of the EAP may be determined by quantifying the reduced mediator.

In the quantitation method of EAP, the oxidoreductase is an oxidase, and the concentration of the EAP may be determined by allowing the oxidoreductase to act on the EA and quantifying generated hydrogen peroxide.

In the quantitation method of EAP, the concentration of the EAP may be determined by allowing the oxidase to act and quantifying generated hydrogen peroxide or consumed oxygen.

In the quantitation method of EAP, a first oxidoreductase allowed to act on the EA, and a second oxidoreductase allowed to act on the at least one kind of amine may be contained.

In the quantitation method of EAP, at least one of the first oxidoreductase and the second oxidoreductase may be an oxidase.

According to an embodiment of the present invention, there is provided an oxidoreductase selected from oxidoreductases belonging to EC NO. 1.4 or EC NO. 1.5, and the oxidoreductase is used in the quantitation method of EAP.

According to an embodiment of the present invention, there is provided an oxidoreductase selected from primary amine dehydrogenase, monoamine dehydrogenase, diamine dehydrogenase, polyamine dehydrogenase, ethanolamine dehydrogenase, tyramine dehydrogenase, phenylethylamine dehydrogenase, benzylamine dehydrogenase, histamine dehydrogenase, serotonin dehydrogenase, spermidine dehydrogenase, spermidine dehydrogenase, β-alanine dehydrogenase, γ-aminobutyric acid (GABA) dehydrogenase, taurine dehydrogenase, cadaverine dehydrogenase, and agmatine dehydrogenase and used in the quantitation method of EAP.

According to an embodiment of the present invention, there is provided an oxidase selected from primary amine oxidase, monoamine oxidase, diamine oxidase, polyamine oxidase, ethanolamine oxidase, tyramine oxidase, phenylethylamine oxidase, benzylamine oxidase, histamine oxidase, serotonin oxidase, spermine oxidase, spermidine oxidase, β-alanine oxidase, γ-aminobutyric acid (GABA) oxidase, taurine oxidase, cadaverine oxidase, and agmatine oxidase and used in the quantitation method of EAP.

According to an embodiment of the present invention, there is provided a quantitation composition for EAP containing a phosphatase and an oxidoreductase acting on the EA.

The quantitation composition for EAP may further include a mediator reduced by adding an oxidoreductase and a reagent reacting with the reduced mediator.

In the quantitation composition for EAP, the oxidoreductase may be an oxidase, and may further include a reagent reacting with hydrogen peroxide generated by adding the oxidase.

According to an embodiment of the present invention, there is provided a quantitation kit for EAP including a phosphatase, and an oxidoreductase acting on the EA.

In the quantitation kit for EAP, a first reagent containing an oxidoreductase and a second reagent containing a mediator reduced by adding a dehydrogenase may be included.

In the quantitation kit for EAP, a first reagent containing an oxidase as the oxidoreductase and a second reagent containing a reagent reacting with hydrogen peroxide generated by adding an oxidase may be included.

In the quantitation kit for EAP, the kit may further include a third reagent containing an oxidoreductase or an oxidase acting on at least one kind of amine other than the EAP, and the third reagent may be added to a sample before adding the first reagent and the second reagent.

According to an embodiment of the present invention, there is provided a sensor chip including a working electrode, a counter electrode, a reference electrode, and a reaction layer arranged on the working electrode, the counter electrode, and the reference electrode, wherein the reaction layer includes an oxidoreductase acting on the EA, and the sensor chip is used in the quantitation method of EAP.

According to an embodiment of the present invention, there is provided a sensor including the sensor chip described above.

### ADVANTAGEOUS EFFECT OF INVENTION

According to an embodiment of the present invention, there is provided a quantitation method of EAP capable of measuring trace amounts of EAP. Alternatively, according to an embodiment of the present invention, there is provided an oxidoreductase for quantitation used in the quantitation method. Alternatively, according to an embodiment of the present invention, there is provided a quantitation composition used in the quantitation method. Alternatively, according to an embodiment of the present invention, there is provided a quantitation kit used in the quantitation method. Alternatively, according to an embodiment of the present invention, there is provided a sensor chip used in the quantitation method. Alternatively, according to an embodiment of the present invention, there is provided a sensor used in the quantitation method.

### BRIEF DESCRIPTION OF DRAWINGS

FIG. 1 is a flowchart illustrating a quantitation method of EAP according to an embodiment of the present invention.
FIG. 2 is a flowchart illustrating a quantitation method of EAP according to an embodiment of the present invention.
FIG. 3 is a flowchart illustrating a quantitation method of EAP according to an embodiment of the present invention.
FIG. 4(a) is a schematic diagram of a sensor chip 10 according to an embodiment of the present invention, and FIG. 4(b) to FIG. 4(d) are schematic diagrams showing members constituting the sensor chip 10.
FIG. 5(a) is a schematic diagram of a sensor 100 according to an embodiment of the present invention, and FIG. 5(b) is a block diagram of a sensor 100 according to an embodiment of the present invention.
FIG. 6 is a diagram showing the reactivity of an EAP (ALP (-) in the figure) or an EAP reacted with ALP (ALP (+) in the figure) with LrHP according to an example of the present invention.
FIG. 7(a) is a diagram showing a measurement result of an EAP sample containing contaminating amines to which 10 µl of water was added instead of ALP, FIG. 7(b) is a measurement result of a sample to which 10 µl of ALP was added to an EAP sample containing contaminating amines according to an example of the present invention.
FIG. 8 is a diagram showing a measurement result of an EAP by changing the EAP concentration in the sample of an example of the present invention to 1.0 µM, 1.5 µM, 3.0 µM, and 5.0 µM.
FIG. 9 is a diagram showing a measurement result of an EAP of an example of the present invention in which AgPEAOX is combined with ALP, and showing a measurement result of an EAP by changing the EAP concentration in the sample to 0.2 mM, 0.5 mM, 1.0 mM.

### DESCRIPTION OF EMBODIMENTS

Hereinafter, a quantitation method of EAP, an oxidoreductase for quantitation, a quantitation composition, a quantitation kit, a sensor chip, and a sensor according to the present invention will be described. However, the quantitation method of EAP, the oxidoreductase for quantitation, the quantitation composition, the quantitation kit, the sensor chip, and the sensor are not to be construed as being limited to the descriptions of the embodiments and examples shown below.

At the time of filing the present application, an enzyme capable of directly quantifying EAP with high sensitivity is not known as a known technique. The present inventors have studied and found that highly sensitive quantitation of EAP can be performed by combining a phosphatase capable of converting an EAP into an EA and an oxidoreductase acting on the EA.

The quantitation method of EAP according to the present invention includes, (1) a step of allowing phosphatase to act on a sample to convert an EAP contained in the sample into an EA, and (2) a step of allowing an oxidoreductase to act on the converted EA.

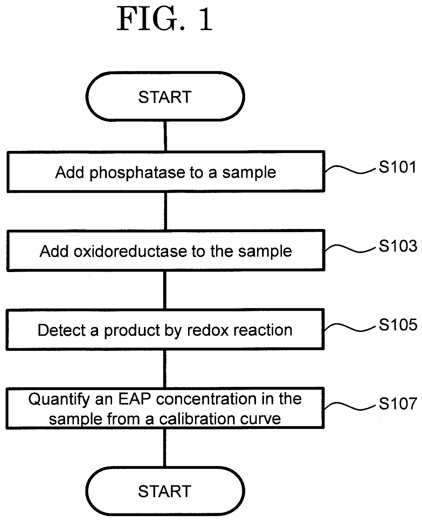

FIG. 1 is a flowchart illustrating a quantitation method of EAP according to an embodiment of the present invention. First, phosphatase is added to the sample and the phosphatase is allowed to act on the EAP to convert it into an EA (S101). An oxidoreductase is added to the sample, and the oxidoreductase is allowed to act on the converted EA (S103). A reaction product produced by this reaction is detected (S105). The EAP is quantified from a detected value of a reaction product using a calibration curve prepared in advance (S107).

In step S101, a concentration of EAP in the sample is not particularly limited, but may be, for example, 0.01 µM to 10000 µM. The pH when performing the phosphatase treatment condition of the sample may be unadjusted or adjusted, the pH may be adjusted to pH 2 to 13, preferably pH 3 to 12, using a suitable pH buffering agent so as to have a suitable pH for the action of the phosphatase to be used. Examples of the buffering agent which can be used include N-[tris(hydroxymethyl)methyl]glycine, phosphate, acetate, carbonate, tris(hydroxymethyl)-aminomethane, borate, citrate, dimethyl glutamate, tricine, HEPES, MES, Bis-Tris, ADA, PIPES, ACES, MOPSO, BES, MOPS, TES, DIPSO, TAPSO, POPSO, HEPPSO, EPPS, Tricine, Bicine, TAPS, phthalic acid, tartaric acid, and the like. A treatment temperature may be, for example, 20°C to 50°C, or it may be performed at a higher temperature range of 45°C to 70°C depending on the phosphatase used. A treatment time may be sufficient to release EA, and may be 5 seconds to 180 minutes, preferably 0.5 minutes to 60 minutes, more preferably 1 minute to 30 minutes, and still more preferably 1 minute to 10 minutes. The obtained treated liquid may be used as it is or heated, centrifuged, concentrated, diluted, or the like as necessary.

In step S103, a duration of action of the oxidoreductase can be, for example, 5 seconds to 180 minutes, preferably 0.5 minutes to 60 minutes, more preferably 1 minute to 30 minutes, and still more preferably 1 minute to 10 minutes. Although a working temperature depends on the optimum temperature of the oxidoreductase to be used, and is, for example, 20°C to 45°C, and the temperature used for the ordinary enzyme reaction can be appropriately selected.

Suitable amounts of oxidoreductase acting on the EA used in the present invention may be added, for example, such that the final concentration is 0.001 U/ml to 500 U/ml, preferably 0.01 U/ml to 100 U/ml. Generally, the lower the concentration of the substrate contained in the sample solution, the higher the final concentration of oxidoreductase to be added. A pH at the time of acting is preferably adjusted by using a buffering agent so as to have a pH suitable for the reaction, considering an optimum pH of oxidoreductase, but is not limited thereto as long as it can act. An example is pH 3 to pH 11, preferably pH 5 to pH 9. Examples of the buffering agent which can be used include, for example, N-[tris(hydroxymethyl)methyl]glycine, phosphate, acetate, carbonate, tris(hydroxymethyl)-aminomethane, borate, citrate, dimethyl glutamate, tricine, HEPES, MES, Bis-Tris, ADA, PIPES, ACES, MOPSO, BES, MOPS, TES, DIPSO, TAPSO, POPSO, HEPPSO, EPPS, Tricine, Bicine, TAPS, phthalic acid, tartaric acid, and the like.

In step S105, a mediator is reduced by oxidoreductase acting on EA and the reduced mediator is reacted with a coloring reagent or color fading reagent. In addition to DCIP (2,6-Dichlorophenolindophenol), examples of a coloring substrate or color fading substrate include, for example, tetrazolium compounds (Tetrazolium blue, Nitro-tetrazolium blue, Water soluble tetrazolium (WST)-1 and WST-3, WST-4, WST-5, WST-8, WST-9), and the like.

In step S105, hydrogen peroxide is produced by the oxidase acting on EA, and the hydrogen peroxide produced by utilizing the catalytic reaction of peroxidase is reacted with a coloring reagent. The coloring reagent or color fading reagent used in the present invention includes, for example, ADPS (N-ethyl-N-sulfopropyl-3-methoxyaniline), ALPS (N-ethyl-N-sulfopropylaniline), TOPS (N-ethyl-N-sulfopropyl-3-methylaniline), ADOS (N-ethyl-N-(2-hydroxy-3-sulfopropyl)-m-anisidine), DAOS (N-ethyl-N-(2-hydroxy-3-sulfopropyl)-3,5-dimethoxyaniline), ALOS (N-ethyl-N-(2-hydroxy-3-sulfopropyl)aniline), HDAOS (N-(2-hydroxy-3-sulfopropyl)-3,5-dimethoxyaniline), MAOS (N-ethyl-N-(2-hydroxy-3-sulfopropyl)-3,5-dimethylaniline), TOOS (N-ethyl-N-(2-hydroxy-3-sulfopropyl)-m-toluidine sodium), DA-67 (10-(carboxymethylaminocarbonyl)-3,7-bis(dimethylamino)-phenocyazine), DA64 (N-(carboxymethylaminocarbonyl)-4,4'-bis(dimethylamino)-diphenylamine), and the like. ADOS, ALOS, TOOS develop colors when condensed with 4-aminoantipyrine. DA-64 and DA-67 do not require 4-aminoantipyrine and develops color only when formulated alone. Further, the coloring reagent includes, but is not limited to, methylene blue, 10-(acetylaminocarbonyl)-3,7-bis(dimethylamino)phenothiazine, 10-(carboxymethylaminocarbonyl)-3,7-bis(dimethylamino)phenothiazine, 10-(phenylcarbonyl)-3,7-bis(dimethylamino)phenothiazine, 10-(methylcarboxyamino)-hexamethyl-amino)-phenothiazine, 10-(3-(methylcarboxyamino)-hexamethyl-amino)-phenothiazine, 10-(3-(methylcarboxyamino)-4-methyl-phenyl)-amino)-phenothiazine, 10-((3-(methylcarboxyaminomethyl)-phenyl)-methylamino)-phenothiazine, 10-(1-naphthalenamino)-phenothiazine, 10-(methyl)-phenothiazine, 10-(phenylamino)-phenothiazine, 10-(methylamino)-phenothiazine, Azure A, Azure B, Azure C, toluidine blue O, 1,9-dimethyl-3,7-bis(dimethylamino)phenothiazine salt, methylene green or a salt thereof, or a leuco form thereof. The coloring reagent may be added such that the final concentration in a reaction solution is 0.001 mM to 10 mM, e.g., 0.005 mM to 2 mM.

In step S105, the amount of EAP in the sample is determined from a detected value of a reaction product using a calibration curve prepared in advance. In this way, EAP in the sample can be quantified.

The sample used in the quantitation method of EAP according to the present invention may be any biological sample that may contain EAP, for example, a sample derived from blood, plasma, interstitial fluid, urine, tears, sweat, saliva, etc. The sample may be processed as appropriate. For example, the sample may be concentrated by a centrifugal concentrator.

Also, in such a quantitation method, in the case where the sample containing EAP is also contaminated with a substance other than EAP on which the oxidoreductase for quantitation of EAP can act, there is a possibility that the measured value may cause an error in a positive direction. In particular, the higher the ratio of oxidoreductase acting on such contaminants, the more difficult it becomes to accurately quantify EAP, which is the original target substance for quantitation.

In an embodiment, the sample may contain at least one kind of amine other than EAP. Generally, a clinical specimen may contain amines such as EA, β-alanine, taurine, and the like as contaminants. In the quantitation method of EAP according to the present invention, since an oxidoreductase acting on EA is used, when these amines are included in the sample, the oxidoreductase also acts on these amines, making it difficult to accurately quantify EA derived from the EAP to be originally measured. Therefore, amines other than EAP contained in the sample are preferably decomposed or eliminated.

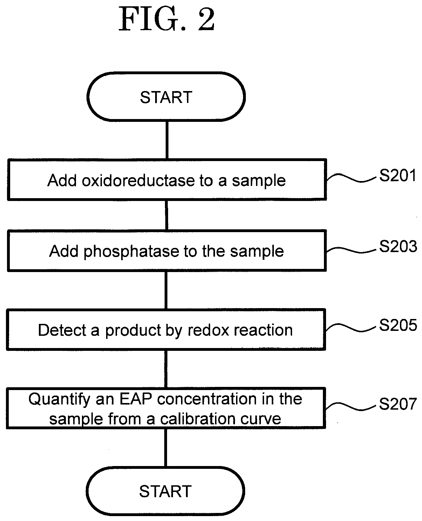

In an embodiment, oxidoreductase acting on at least amines is preferably allowed to act on the amine contained in the sample before allowing phosphatase to act on the sample. FIG. 2 is a flowchart illustrating a quantitation method of EAP according to an embodiment of the present invention. First, oxidoreductase is added to a sample, and amines contained in the sample are decomposed or eliminated (S201). Phosphatase is added to the sample and allowed to act on EAP to convert it into EA (S203). The oxidoreductase added in step S201 acts on the converted EA. The reaction product produced by this reaction is detected (S205). EAP is quantified from a detected value of a reaction product using a calibration curve prepared in advance (S207).

In step S201, since the oxidoreductase acting on at least amines is first added to the sample, the amine contained in the sample is decomposed or eliminated by the oxidoreductase. The degree of effect of the error-causing substance in the sample on the measured value in the quantitation method of EAP according to the present invention depends on the amount of amines such as free EA and the like contained in the sample. In the case where a sample having a small amount of amines such as free EA contained in the sample is measured, a step S201 of eliminating amines such as free EA in advance is not required, and phosphatase can be allowed to directly act on the sample. In that case, it can be inferred that all EA produced in the sample was excised from EAP in the sample by the action of phosphatase.

On the other hand, in the case where a sample containing amines such as free EA is measured, it is not possible to distinguish EAP from EA liberated by the action of phosphatase, which is the target material for quantitation, resulting in a measurement error. In order to avoid this measurement error, it is preferred to eliminate amines such as free EA in the sample in advance.

Also, in the case of selecting an oxidoreductase which has a high specificity for EA and does not substantially act on EAP, the step S201 does not eliminate EAP, which is the target substance for quantitation. However, if the property of the oxidoreductase used is such that it eliminates EAP, which is the target substance for quantitation in the step S201, erroneous elimination of EAP can be avoided by setting a conditional concentration in which EAP is not eliminated and only amines such as EA are eliminated, and performing the step S201.

In step S203, phosphatase is added to the sample and allowed to act on EAP to convert it into EA. Since the step S203 is the same step as the step S101 described above, a detailed explanation thereof will be omitted.

In step S205, the oxidoreductase added in step S201 acts on the EA converted in step S203. The reaction product produced by this reaction can be detected. Since the step S205 is the same as that of the step S103 except that the oxidoreductase already added acts on EA, and is the same as that of the step S105 in that the reaction product produced in the redox reaction is detected, a detailed explanation thereof will be omitted.

In step S207, EAP is quantified from a detected value of a reaction product using a calibration curve prepared in advance. Since the step S207 is the same as the step S107, a detailed explanation thereof will be omitted.

In the embodiment described above, although an example is shown in which the action of the oxidoreductase added in the amine-elimination step is used as it is, the quantitation method of EAP is not limited to this. An oxidoreductase may be added at the time of the amine-elimination step, and the oxidoreductase may be further added in the EAP quantitation step.

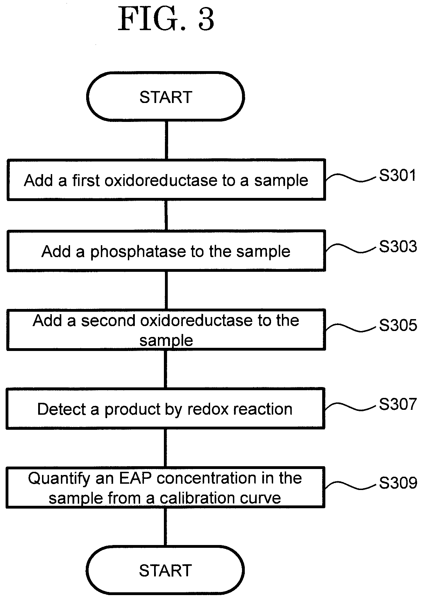

FIG. 3 is a flowchart illustrating a quantitation method of EAP according to an embodiment of the present invention. First, a first oxidoreductase is added to a sample, and amines contained in the sample are decomposed or eliminated (S301). Phosphatase is added to the sample and allowed to act on EAP to convert it into EA (S303). A second oxidoreductase is added to the sample and allowed to act on the converted EA (S305). The reaction product produced by this reaction is detected (S307). EAP is quantified from a detected value of a reaction product using a calibration curve prepared in advance (S309).

The oxidoreductase used for eliminating amines such as free EA in the sample in step S301 and the oxidoreductase used in step S305 for quantifying EA released by the action of the phosphatase from EAP, which is the target substance for quantitation, may be different types, or the same type. In addition, an oxidase described later may be used as at least one oxidoreductase used for eliminating amines such as free EA in a sample and oxidoreductase used for quantifying EA released by the action of phosphatase from EAP, which is a target substance for quantitation. Also, a concentration of the enzyme suitable for use in elimination, a concentration of the enzyme suitable for use in quantitation, or a substrate specificity of the enzyme to be used can be appropriately set.

The step S303 may be the same step as the step S101 described above. The step S305 may be the same step as the step S103 described above. The step S307 may be the same step as the step S105 described above. The step S309 may be the same step as the step S107 described above, and a detailed explanation thereof will be omitted.

### <Phosphatase>

Any phosphatase may be used as the phosphatase that can be used in the quantitation method of EAP as long as it can be used in a laboratory test and can effectively excise an EA from the EAP in a sample to be processed. Examples of such phosphatases include acid phosphatases, alkaline phosphatases, and protein phosphatases. The phosphatase may be used alone or in a combination of two or more.

In the case where the phosphatase is selected in the present invention, the enzyme can be selected from the viewpoint of suppressing the generation of contaminants as much as possible, and at the same time efficiently cutting out EA from the EAP in the sample. That is, it is possible to suppress the generation of contaminants by predicting various compounds to which phosphoric acid is added other than the measurement object as contaminants contained in the sample, and selecting a type of phosphatase that is difficult to produce them. Phosphatases can be selected based on, for example, knowledge of what types of bonds between structures are likely to be cleaved by various known phosphatases.

In practice, the quantitation method of EAP according to the present invention can also be controlled by selecting the substrate specificity of the oxidoreductase in the step of the action by the oxidoreductase following cleavage by the phosphatase, and such selection is expected to reduce the effects of erroneous reactions.

The phosphatase treatment condition of the sample may be any condition as long as the phosphatase used acts on EAP and efficiently liberates EA in a short time. The amount of phosphatase to be used is appropriately selected depending on the content of EAP contained in the sample, treatment conditions, and the like, and as an example, the phosphatase can be added so that the final concentration is 0.001 U/ml to 10000 U/ml, preferably 0.01 U/ml to 1000 U/ml. Further, other enzymes may be added as appropriate.

The activity of the phosphatase that can be used in the present invention is calculated by measuring the amount of 4-nitrophenol (maximum absorbance wavelength of 405 nm) produced by hydrolysis of 4-nitrophenyl phosphate using a spectrophotometer. As an example, when using alkaline phosphatase (ALP), the amount of enzyme that hydrolyzes 1 µmol of 4-nitrophenyl phosphate per minute at 37°C with pH 9.8 (diethanolamine buffer) was defined as 1U.

### <Oxidoreductase>

Oxidoreductases that can be used in the present invention are enzymes that act on an EA or a substrate structurally similar to EA. A dehydrogenase that efficiently acts on a substrate having a similar structure to EA can be used as a substitute for the oxidoreductase used in the present invention. The similar structure refers to a physicochemical structure that is considered similar from a structural, electronic, stereochemical point of view, and the like.

For example, substrates structurally similar to EA include substrates having a CH-NH₂ bond or CH-NH bond, and the enzymes for the substrates include oxidoreductases belonged to EC NO. 1.4 or EC NO. 1.5.

For example, oxidoreductases belonging to EC NO. 1.4 or 1.5 include primary amine dehydrogenase, monoamine dehydrogenase, diamine dehydrogenase, polyamine dehydrogenase, ethanolamine dehydrogenase, tyramine dehydrogenase, phenylethylamine dehydrogenase, benzylamine dehydrogenase, histamine dehydrogenase, serotonin dehydrogenase, spermine dehydrogenase, spermidine dehydrogenase, β-alanine dehydrogenase, γ-aminobutyric acid (GABA) dehydrogenase, taurine dehydrogenase, cadaverine dehydrogenase, agmatine dehydrogenase. In particular, a substrate structurally similar to EAP is taurine, and taurine dehydrogenase (TDH) can be suitably used as a dehydrogenase for the substrate.

The oxidoreductase used in the present invention may be a multimer or a monomer. For example, if only a certain subunit (monomer) among several subunits constituting oxidoreductase which is a multimer catalyzes the dehydrogenation reaction in which hydrogen is taken from the substrate and transferred to a hydrogen acceptor, the oxidoreductase used in the present invention may be a multimer or the subunit (monomer).

In an embodiment, the oxidoreductase used in the present invention has a large subunit (LaTDH) having the base sequence of SEQ ID NO: 1 and a small subunit (SmTDH) having the base sequence of SEQ ID NO: 2, and catalyzes a dehydrogenation reaction in which not only a multimer having a LaTDH and an SmTDH but also only a LaTDH takes hydrogen from a substrate and transfers it to a hydrogen acceptor. Therefore, the oxidoreductase used in the present invention may have LaTDH and SmTDH, or may be LaTDH alone.

In addition, the oxidoreductases belonging to EC NO. 1.4 or EC NO. 1.5 may be oxidases belonging to EC NO. 1.4.3 or EC NO. 1.5.3.

For example, oxidases belonging to EC NO. 1.4.3 or EC NO. 1.5.3 include primary amine oxidase, monoamine oxidase, diamine oxidase, polyamine oxidase, ethanolamine oxidase, tyramine oxidase, phenylethylamine oxidase, benzylamine oxidase, histamine oxidase, serotonin oxidase, spermine oxidase, spermidine oxidase, β-alanine oxidase, γ-aminobutyric acid (GABA) oxidase, taurine oxidase, cadaverine oxidase, and agmatine oxidase. In particular, phenylethylamine oxidase (PEAOX) can be suitably used.

Any conditions may be used as the reaction condition of the oxidoreductase used in the quantitation method of EAP according to the present invention as long as the condition acts on EA and efficiently catalyzes the oxidation reaction. However, enzymes generally have an optimum temperature and pH at which the enzymes exhibit the highest activity. Therefore, the reaction condition is preferably near the optimum temperature and the optimum pH. For example, a temperature of 30°C and pH8.5, described later, are suitably used as oxidoreductase reaction conditions but are not limited to this. For example, a temperature of 37°C and pH 8.5, described later, are suitably used as a reaction condition of PEAOX but are not limited to this.

The oxidoreductase used in the quantitation method of EAP according to the present invention may be oxidoreductase produced by a naturally occurring microorganism or oxidoreductase produced by a transformed microorganism. From the viewpoint of efficient mass expression of the enzyme, the enzyme can be efficiently mass-expressed by using the transformed microorganism.

Microorganisms from which the oxidoreductase used in the quantitation method of EAP according to the present invention is derived are not particularly limited, but microorganisms belonging to the genus Paracoccus, the genus Methylarcula, the genus Martelella, the genus Rhodobacter, the genus Roseobacter, the genus Gemmobacter, the genus Arthrobacter, the genus Paenarthrobacter, the genus Pseudarthrobacter, the genus Cryobacterium, the genus Bacillus, the genus Sinomonas, the genus Tersicoccus, the genus Kocuria, the genus Micrococcus, the genus Brevibacterium, the genus Zhihengliuella, the genus Citricoccus, the genus Geodematophilus, the genus Rhodococcus, the genus Amycolatopsis, the genus Nocardia, the genus Modestobacter, the genus Glutamicibacter, the genus Psudonocardia, the genus Gordonia, the genus Streptomyces, the genus Geodermatophilus, the genus Cellulomonas, the genus Mycobacterium, the genus Mycolicibacterium, the genus Psudoglutamicibacter, the genus Corynebacterium, the genus Nocardiopsis, the genus Nonomuraea, the genus Saccharomonospora, the genus Prauserella, the genus Amnibacterium, the genus Actinobacteria, the genus Saccharopolyspora, the genus Leifsonia, the genus Agromyces, the genus Streptacidiphilus, the genus Xylanimonas, the genus Tsukamurella, the genus Williamsia, the genus Asanoa, the genus Plantactinospora, the genus Salinispora, the genus Agreia, the genus Cryocola, the genus Curtobacterium, the genus Murinocardiopsis, the genus Subtercola, the genus Microbispora, the genus Jiangella, the genus Blastococcus, the genus Actinomadura, the genus Actinoplanes, the genus Catenulispora, the genus Lichtheimia, the genus Syncephalastrum can be preferably used.

For example, although the oxidoreductase used in the quantitation method of EAP according to the present invention may be THD produced by Paracoccus denitrigicans or TDH produced by E. coli transformed with a plasmid containing a TDH gene derived from Paracoccus denitrigicans, oxidoreductase can be efficiently expressed in large quantities by using the E. coli transformed with the plasmid containing the TDH gene derived from Paracoccus denitrigicans.

In addition, for example, although the oxidoreductase used in the quantitation method of EAP according to the present invention may be a PEAOX produced by Arthrobacter globiformis or a PEAOX produced by the E. coli transformed with a plasmid containing a PEAOX gene derived from Arthrobacter globiformis, oxidoreductase can be efficiently expressed in large quantities by using the E. coli transformed with the plasmid containing the PEAOX gene derived from Arthrobacter globiformis.

In addition, for example, although the oxidoreductase used in the quantitation method of EAP according to the present invention may be amine oxidase (LcAOX) produced by Lichtheimia corymbifera or amine oxidase produced by E. coli transformed with a plasmid containing a LcAOX gene having a base sequence of SEQ ID NO: 3 derived from Lichtheimia corymbifera, oxidoreductase can be efficiently expressed in large quantities by using the E. coli transformed with the plasmid containing the LcAOX gene derived from Lichtheimia corymbifera.

In addition, for example, although the oxidoreductase used in the quantitation method of EAP according to the present invention may be hypothetical protein (LrHP) produced by Lichtheimia ramosa or hypothetical protein produced by E. coli transformed with a plasmid containing an LrHP gene having a base sequence of SEQ ID NO: 4 derived from Lichtheimia ramosa, oxidoreductase can be efficiently expressed in large quantities by using the E. coli transformed with the plasmid containing oxidoreductase derived from Lichtheimia ramosa.

In addition, for example, although the oxidoreductase used in the quantitation method of EAP according to the present invention may be amine oxidase (SrAOX3925) produced by Syncephalastrum racemosum or amine oxidase produced by E. coli transformed with a plasmid containing an SrAOX3925 gene having a base sequence of SEQ ID NO: 5 derived from Syncephalastrum racemosum, oxidoreductase can be efficiently expressed in large quantities by using the E. coli transformed with the plasmid containing the SrAOX3925 gene derived from Syncephalastrum racemosum.

In addition, for example, although the oxidoreductase used in the quantitation method of EAP according to the present invention may be amine oxidase (SrAOX3926) produced by Syncephalastrum racemosum or amine oxidase produced by E. coli transformed with a plasmid containing an SrAOX3926 gene having a base sequence of SEQ ID NO: 6 derived from Syncephalastrum racemosum, oxidoreductase can be efficiently expressed in large quantities by using the E. coli transformed with the plasmid containing the SrAOX3926 gene derived from Syncephalastrum racemosum.

In addition, for example, although the oxidoreductase used in the quantitation method of EAP according to the present invention may be ethanolamine oxidase (SrEAOX) produced by Syncephalastrum racemosum or ethanolamine oxidase produced by E. coli transformed with a plasmid containing an SrEAOX gene having a base sequence of SEQ ID NO: 7 derived from Syncephalastrum racemosum, oxidoreductase can be efficiently expressed in large quantities by using the E. coli transformed with the plasmid containing the SrEAOX gene derived from Syncephalastrum racemosum.

In an embodiment, the oxidoreductase used in the quantitation method of EAP according to the present invention includes oxidoreductase which has a high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to the amino acid sequence (SEQ ID NO: 8) of LaTDH produced by Paracoccus denitrigicans and oxidoreductase having an amino acid sequence in which one or more amino acids have been altered or varied, deleted, substituted, added and/or inserted in the amino acid sequence of SEQ ID NO: 8.

In an embodiment, the oxidoreductase used in the quantitation method of EAP according to the present invention includes oxidoreductase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to an amino acid sequence (SEQ ID NO: 9) of PEAOX produced by Arthrobacter globiformis, and oxidoreductase having an amino acid sequence in which one or more amino acids are altered or varied, deleted, substituted, added and/or inserted in the amino acid sequence of SEQ ID NO: 9.

In an embodiment, the oxidoreductase used in the quantitation method of EAP according to the present invention includes oxidoreductase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to an amino acid sequence (SEQ ID NO: 10) of LcAOX produced by Lichtheimia corymbifera, and oxidoreductase having an amino acid sequence in which one or more amino acids are altered or varied, deleted, substituted, added and/or inserted in the amino acid sequence of SEQ ID NO: 10.

In an embodiment, the oxidoreductase used in the quantitation method of EAP according to the present invention includes oxidoreductase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to an amino acid sequence (SEQ ID NO: 11) of LrHP produced by Lichtheimia ramosa, and oxidoreductase having an amino acid sequence in which one or more amino acids are altered or varied, deleted, substituted, added and/or inserted in the amino acid sequence of SEQ ID NO: 11.

In an embodiment, the oxidoreductase used in the quantitation method of EAP according to the present invention includes oxidoreductase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to an amino acid sequence (SEQ ID NO: 12) of SrAOX3925 produced by Syncephalastrum racemosum, and oxidoreductase having an amino acid sequence in which one or more amino acids are altered or varied, deleted, substituted, added and/or inserted in the amino acid sequence of SEQ ID NO: 12.

In an embodiment, the oxidoreductase used in the quantitation method of EAP according to the present invention includes oxidoreductase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to an amino acid sequence (SEQ ID NO: 13) of SrAOX3926 produced by Syncephalastrum racemosum, and oxidoreductase having an amino acid sequence in which one or more amino acids are altered or varied, deleted, substituted, added and/or inserted in the amino acid sequence of SEQ ID NO: 13.

In an embodiment, the oxidoreductase used in the quantitation method of EAP according to the present invention includes oxidoreductase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to an amino acid sequence (SEQ ID NO: 14) of SrEAOX produced by Syncephalastrum racemosum, and oxidoreductase having an amino acid sequence in which one or more amino acids are altered or varied, deleted, substituted, added and/or inserted in the amino acid sequence of SEQ ID NO: 14.

### (Amino Acid Sequence Identity)

Amino acid sequence identity can be calculated by programs such as maxim matching or search homology of GENETYX (registered trademark) (GENETYX CORPORATION), or maxim matching or multiple alignment of DNASIS (registered trademark) Pro (Hitachi Solutions, Ltd.), or multiple alignment of CLUSTAL W When the amino acid sequences of two or more oxidoreductases are aligned in order to calculate the amino acid sequence identity, a position of the amino acids which is identical in the 2 or more oxidoreductases can be examined. An identical region in the amino acid sequence can be determined based on such information. Here, with respect to two or more amino acid sequences, percent identity refers to a percentage with the total number of amino acids in the region where the amino acids can be aligned as a denominator and the number of positions occupied by the identical amino acid as a numerator when the two or more amino acid sequences are aligned using algorithms such as Blosum62. Therefore, in general, when there is a region in which no identity is found in two or more amino acid sequences, for example, when an additional sequence in which no identity is found at the N-terminus or the C-terminus exists in one amino acid sequence, the region in which no identity is found cannot be aligned, and therefore, it is not used to calculate the percent identity.

### (Method for Preparing Enzyme)

Hereinafter, a method for preparing oxidoreductase used in the quantitation method of EAP according to the present invention will be described.

### (Construction of Expression Plasmid)

The plasmid for expression oxidoreductase used in the quantitation method of EAP according to the present invention is obtained by a commonly used method. For example, DNA is extracted from a microorganism producing oxidoreductase to construct a DNA library. A DNA fragment encoding the oxidoreductases according to the present invention is identified and isolated from the constructed DNA library. The DNA fragment is amplified by a polymerase chain reaction (PCR) with complementary primers in which the isolated DNA fragment is used as a template to clone a gene encoding the oxidoreductase. The amplified DNA fragment is ligated into a vector to obtain a plasmid having the DNA fragment encoding the oxidoreductase.

Alternatively, the DNA fragment encoding the oxidoreductase is chemically synthesized, and the DNA fragment is ligated into the vector to obtain the plasmid having DNA encoding the oxidoreductase.

A strain such as E. coli is transformed with the obtained plasmid to obtain a strain such as the E. coli having the DNA encoding the oxidoreductase.

Further, for example, the strain such as yeast may be transformed with the obtained plasmid to obtain a strain such as yeast having the DNA encoding oxidoreductase. As a transformation method to yeast, a known method, for example, a method using lithium acetate (Methods Mol. Cell. Biol., 5, 255-269 (1995)), an electroporation (J Microbiol Methods 55 (2003) 481-484), or the like, can be suitably used, but the present invention is not limited thereto, and transformation may be performed using various optional techniques including a spheroplast method, a glass bead method, and the like. Microorganisms classified as yeast include, for example, yeasts belonging to the genus Zygosaccharomyces, the genus Saccharomyces, the genus Pichia, and the genus Candida. The plasmid having the DNA encoding the oxidoreductase may include a marker gene to allow the selection of transformed cells. The marker gene includes, for example, genes which complement the auxotrophy of the host, such as URA3, TRP1. It is also desirable that the plasmid having the DNA encoding the oxidoreductase contains a promoter or other control sequence (e.g., secretory signal sequence, enhancer sequence, terminator sequence or polyadenylation sequence, and the like) capable of expressing the oxidoreductase gene of the present invention in the host cell. Specific examples of the promoter include a GAL1 promoter, and an ADH1 promoter, and the like.

Other examples of host cells include, for example, filamentous fungi such as the genus Aspergillus and the genus Tricoderma. A method for producing a transformant of a filamentous fungus is not particularly limited, and examples thereof include a method of inserting into a host filamentous fungus in an aspect in which the DNA encoding the oxidoreductase is expressed according to a conventional method. Specifically, a transformant overexpressing the gene encoding oxidoreductase is obtained by making a DNA construct in which the gene encoding the oxidoreductase of the present invention is inserted between an expression-inducing promoter and a terminator, then transforming the host filamentous fungus with the DNA construct containing the gene encoding oxidoreductase. In this specification, the DNA fragment consisting of an expression-inducible promoter-a gene encoding the oxidoreductase-terminator and a recombinant vector containing the DNA fragment produced for transforming a host filamentous fungus are collectively referred to as a DNA construct.

The method of inserting the gene encoding the oxidoreductase into the host filamentous fungus in such a manner that the gene is expressed is not particularly limited, and for example, the method includes a method of inserting the gene directly into the chromosome of the host organism by using homologous recombination, or a method of introducing the gene into the host filamentous fungus by linking on a plasmid vector, and the like.

In a method using homologous recombination, the DNA construct can be ligated between sequences homologous to an upstream region and a downstream region of the recombination site on the chromosome and inserted into the genome of the host filamentous fungus. Transformants by self-cloning can be obtained by overexpressing within the host filamentous fungi under high expression promoter control of the host filamentous fungus itself. The high expression promoter is not particularly limited, and examples thereof include a promoter region of a TEF1 gene (tef1), which is a translational elongation factor, a promoter region of an α-amylase gene (amy), and an alkaline protease gene (alp) promoter region.

In a method using the vector, the DNA construct can be incorporated into the plasmid vector used in the transformation of filamentous fungi by a conventional method, and the corresponding host filamentous fungus can be transformed by a conventional method.

Such a suitable vector-host system is not particularly limited as long as it is a system capable of producing the oxidoreductase of the present invention in a host filamentous fungus, and examples thereof include a system of pUC19 and filamentous fungi, a system of pSTA14 (Mol. Gen. Genet. 218, 99-104, 1989), and filamentous fungi.

The DNA construct is preferably introduced into the chromosomes of the host filamentous fungus and used, in other ways, but can also be used without introduction into the chromosomes by incorporating the DNA construct into an autonomously replicated vector (Ozeki et al. Biosci. Biotechnol. Biochem. 59, 1133 (1995)).

The DNA construct may include a marker gene to allow the selection of transformed cells. The marker gene is not particularly limited, and examples of the marker gene include a gene which complements the auxotrophy of the host, such as pyrG, niaD, adeA; and a drug resistance gene against a drug, such as pyrithiamine, hygromycin B, or oligomycin. It is also preferred that the DNA construct contains a promoter, a terminator, or other control sequences (e.g., an enhancer, a polyadenylation sequence, and the like) which allow for overexpression of the gene encoding the oxidoreductase of the present invention in the host cell. Promoters include, but are not limited to, an appropriate expression-inducing promoter and a constitutive promoter, such as a tef1 promoter, an alp-promoter, an amy-promoter, and the like. The terminator is also not particularly limited, and examples thereof include an alp terminator, an amy terminator, and a tef1 terminator.

In the DNA construct, the expression control sequence of the gene encoding the oxidoreductase is not necessarily required when the DNA fragment including the gene encoding the oxidoreductase of the present invention, which will be inserted, includes a sequence having an expression control function. When transformation is performed by a co-transformation method, the DNA construct may not have to have a marker gene in some cases.

An embodiment of the DNA construct is a DNA construct in which, for example, a tef1 gene promoter, a gene encoding the oxidoreductase, an alp gene terminator, and a pyrG marker gene are ligated to an In-Fusion Cloning Site at a multicloning site of pUC19.

As a transformation method to a filamentous fungus, a method known to those skilled in the art can be appropriately selected, and for example, a protoplast PEG method using polyethylene glycol and calcium chloride after preparing a protoplast of a host filamentous fungus (see, for example, Mol. Gen. Genet. 218, 99-104, 1989, Japanese laid-open patent publication No. 2007-222055, and the like) can be used. As a medium for regenerating the transformed filamentous fungus, an appropriate medium is used according to the host filamentous fungus to be used and the transformation marker gene. For example, when Aspergillus sojae is used as the host filamentous fungus and a pyrG gene is used as the transformation marker gene, regeneration of the transformed filamentous fungus can be performed, for example, in a Czapek-Dox minimal medium (manufactured by Difco Laboratories) containing 0.5% agar and 1.2 M sorbitol.

### (Recombinant Expression of Enzyme)

The strain such as E. coli having the DNA encoding the oxidoreductase used in the quantitation method of EAP according to the present invention is cultured in a medium. When culturing a microbial host cell, it may be carried out by aeration-agitated deep culture, shaking culture, stationary culture, or the like, at a culture temperature of 10°C to 42°C, preferably at a culture temperature of about 25°C, for several hours to several days, and more preferably at a culture temperature of about 25°C, for preferably 1 to 7 days. As the medium for culturing the microorganism host cell, for example, a medium is used in which one or more kinds of inorganic salts such as sodium chloride, monopotassium phosphate, dipotassium phosphate, magnesium sulfate, magnesium chloride, ferric chloride, ferric sulfate, or manganese sulfate are added to one or more kinds of nitrogen sources such as yeast extract, tryptone, peptone, meat extract, corn steep liquor, or leaching solution of soybean or wheat bran, and if necessary, a saccharine material, vitamins, and the like are appropriately added. Bacterial cells are separated from the culture medium obtained by the culturing by centrifugation. The bacterial cells obtained by the separation are subjected to ultrasonic grinding, grinding, or the like, or to treatment with a lytic enzyme such as lysozyme or yatalase to obtain a suspension, and the suspension is centrifuged to obtain a crude enzyme solution from the obtained fraction.

### (Purification of Oxidoreductase)

The method for purifying oxidoreductase may be any method as long as it is capable of purifying an enzyme from a crude enzyme solution. For example, oxidoreductase can be purified from the crude enzyme solution by a commonly used method such as ion exchange chromatography, gel filtration chromatography, or the like.

### (Oxidoreductase Activity Measurement)

The method for measuring the activity of the oxidoreductase may be any method as long as it directly or indirectly measures a product of a redox reaction catalyzed by the oxidoreductase. For example, a reduced product is generated by catalyzing a redox reaction by the oxidoreductase, and a current value generated by the reduced product passing electrons to an electrode is measured. Thus, it is possible to measure the enzyme activity. Suitably, the enzyme activity can be measured by reacting the reduced product by the redox reaction catalyzed by the oxidoreductase with a reagent containing a light-absorbing substance reacting with the reduced product (hereinafter, a "light-absorbing reagent") and performing an absorbance measurement.

### (Composition for Quantification of EAP and Kit for Quantification of EAP)

The quantitation method of EAP utilizing phosphatase and oxidoreductase according to the present invention may be carried out by providing phosphatase and a composition containing oxidoreductase acting on EA, or may be carried out by combining phosphatase, oxidoreductase acting on EA, and a commercially available product reaction reagent. For example, the quantitation method may be provided as a quantitation composition of EAP containing phosphatase and oxidoreductase acting on EA, and as a composition for the quantitation of EAP further including a mediator which is reduced by adding the oxidoreductase and a reagent that reacts with the reduced mediator.

In an embodiment, the quantitation method may be provided as a quantitation kit of EAP including phosphatase, oxidoreductase, a mediator which is reduced by adding the oxidoreductase, and a reagent that reacts with the reduced mediator. For example, the quantitation kit of EAP may include phosphatase, a first reagent containing oxidoreductase, and a second reagent containing a mediator which is reduced by adding oxidoreductase. In addition, it may be configured to include a third reagent including oxidoreductase or oxidase acting on at least one kind of amine other than ethanolamine phosphate, and add the third reagent to the sample before adding the first reagent and the second reagent.

The mediator (also referred to as an artificial electron mediator, an artificial electron acceptor, or an electron mediator) used in the quantitation method or the quantitation kit of the present invention is not particularly limited as long as it can receive electrons from oxidoreductase. Examples of the mediators include quinones, phenazines, viologens, cytochromes, phenoxazines, phenothiazines, ferricyanides, e.g., potassium ferricyanide, ferredoxins, ferrocenes, osmium complexes and derivatives thereof, and the phenazine compounds include, but are not limited to, 5-Methylphenazinium methosulfate (PMS) and methoxy PMS.

### (Sensor Chip and Electrode)

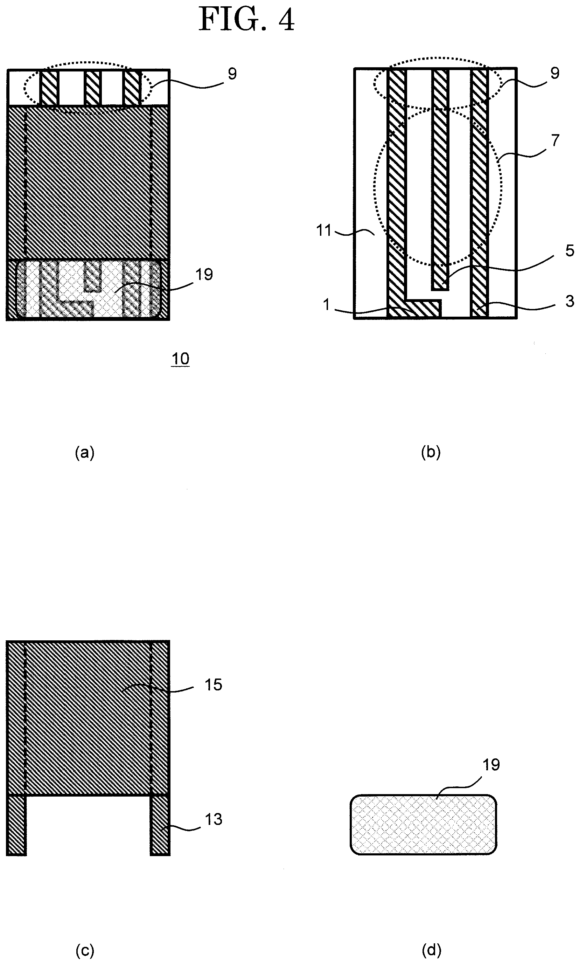

FIG. 4(a) is a schematic diagram of a sensor chip 10 according to an embodiment of the present invention, and FIG. 4(b) to FIG. 4(d) are schematic diagrams showing a member constituting the sensor chip 10. The sensor chip 10 includes two or more electrodes arranged on a substrate 11. The substrate 11 is made of an insulating material. In FIG. 4(a) and FIG. 4(b), as an example, a working electrode 1, a counter electrode 3, and a reference electrode 5 are arranged on the substrate 11. Each electrode is electrically connected to a wiring portion 7, and the wiring portion 7 is electrically connected to a terminal 9 located on the other side of each electrode in the wiring direction. The working electrode 1, the counter electrode 3, and the reference electrode 5 are arranged apart from each other. The working electrode 1, the counter electrode 3, and the reference electrode 5 are preferably formed integrally with the wiring portion 7 and the terminal 9. Further, the counter electrode 3 and the reference electrode 5 may be integral.

As shown in FIG. 4(a) and FIG. 4(c), a spacer 13 is arranged on an end of the substrate 11 which is parallel to the wiring portion 7, and a cover 15 which covers the working electrode 1, the counter electrode 3, the reference electrode 5, and the spacer 13 is arranged. The spacer 13 and the cover 15 are made of an insulating material. The spacer 13 preferably has a thickness substantially equal to that of the working electrode 1, the counter electrode 3, and the reference electrode 5, and is in close contact with the working electrode 1, the counter electrode 3, and the reference electrode 5. In addition, the spacer 13 and the cover 15 may be integrally formed. The cover 15 is a protective layer which prevents the wiring portion 7 from being deteriorated by being exposed to the outside air and short-circuiting due to the penetration of the measurement sample.

In an embodiment, the oxidoreductase of the present invention may be applied, adsorbed, or immobilized on the electrode. Preferably, the oxidoreductase of the present invention is applied, adsorbed, or immobilized on the working electrode. In another embodiment, phosphatase together with oxidoreductase may also be applied, adsorbed, or immobilized on the electrode. In another embodiment, the mediator together with oxidoreductase and phosphatase may also be applied, adsorbed, or immobilized on the electrode. Oxidoreductase, or oxidoreductase and phosphatase, or oxidoreductase and phosphatase and a mediator, may be included in a reaction layer 19 arranged on the working electrode 1, the counter electrode 3, and the reference electrode 5. A carbon electrode, a metal electrode such as platinum, gold, silver, nickel, and palladium, or the like can be used as the electrode. In the case of carbon electrodes, examples of the material include pyrolytic graphite carbon (PG), glassy carbon (GC), carbon paste and plastic formed carbon (PFC), and the like. A measurement system may be a two-electrode system or a three-electrode system, for example, and enzymes may be immobilized on the working electrode. Examples of the reference electrode include a standard hydrogen electrode, a reversible hydrogen electrode, a silver-silver chloride electrode (Ag/AgCl), a palladium-hydrogen electrode, and a saturated calomel electrode, and the Ag/AgC! is preferably used from the viewpoint of stability and reproducibility.

The oxidoreductase can be immobilized on the electrode by crosslinking, coating with a dialysis membrane, encapsulation in a polymer matrix, use of a photocrosslinkable polymer, use of an electrically conductive polymer, use of an oxidization/reduction polymer, and the like. The enzymes may also be immobilized in a polymer or adsorbed onto the electrode together with a mediator, or these techniques may be combined.

The mediator (also referred to as an artificial electron mediator, an artificial electron acceptor or an electron mediator) used in the composition, kit, electrode, or sensor chip of the present invention is not particularly limited as long as it can receive electrons from oxidoreductase. Examples of the mediators include quinones, phenazines, viologens, cytochromes, phenoxazines, phenothiazines, ferricyanides, e.g., potassium ferricyanide, ferredoxins, ferrocenes, osmium complexes and derivatives thereof, and the like, and examples of the phenazine compounds include, but are not limited to, PMS and methoxy PMS.

The oxidoreductase of the present invention can be applied to various electrochemical measurement methods by using a potentiostat, a galvanostat, or the like. The electrochemical measurement includes various techniques such as amperometry, potentiometry and coulometry. For example, by using an amperometry method, the concentration of EAP in a sample can be calculated by measuring a current value generated by applying +600 mV to +1000 mV (vs. Ag/AgCl) by a hydrogen peroxide electrode to hydrogen peroxide produced when oxidoreductase reacts with EAP. For example, a calibration curve can be generated by measuring current values for known concentrations of EAP (0 µM, 0.1 µM, 0.15 µM, 0.3 µM, 0.5 µM, 50 µM, 100 µM, 150 µM, 200 µM) and plotting against concentrations of EAP. The concentration of EAP can be obtained from the calibration curve by measuring the current of the unknown EAP. For example, a carbon electrode or a platinum electrode can be used as the hydrogen peroxide electrode. The amount of hydrogen peroxide can be quantified by measuring the reduction current value generated by applying -400 mV to +100 mV (vs. Ag/AgCl) using an electrode immobilized with a reductase such as peroxidase or catalase, instead of the hydrogen peroxide electrode, and the value of EAP can be measured.

In addition, by using an amperometry method, the concentration of EAP in the sample can be calculated by mixing a mediator in a reaction solution, transferring electrons generated when the oxidase reacts with EAP to an oxidized mediator, generating a reduced mediator, and measuring a current value generated by applying -1000 mV to +500 mV (vs. Ag/AgCl). A carbon electrode or a platinum electrode is preferred as the counter electrode. For example, a calibration curve can be generated by measuring current values for known concentrations of EAP (0 µM, 0.1 µM, 0.15 µM, 0.3 µM, 0.5 µM, 50 µM, 100 µM, 150 µM, 200 µM) and plotting against the concentrations of EAP. The concentration of EAP can be obtained from the calibration curve by measuring the current value of the unknown EAP.

In addition, printed electrodes (sensor chips) can be used to reduce the amount of solution required for measurement. In this case, the electrodes are preferably formed on a substrate composed of an insulating substrate. Specifically, the electrodes are preferably formed on the substrate by photolithography or printing techniques such as screen printing, gravure printing, and flexographic printing. Further, examples of the material of the insulating substrate include silicon, glass, ceramic, polyvinyl chloride, polyethylene, polypropylene, and polyester, but those having strong resistance to various solvents and chemicals are more preferably used.

### [EAP Measurement Sensor]

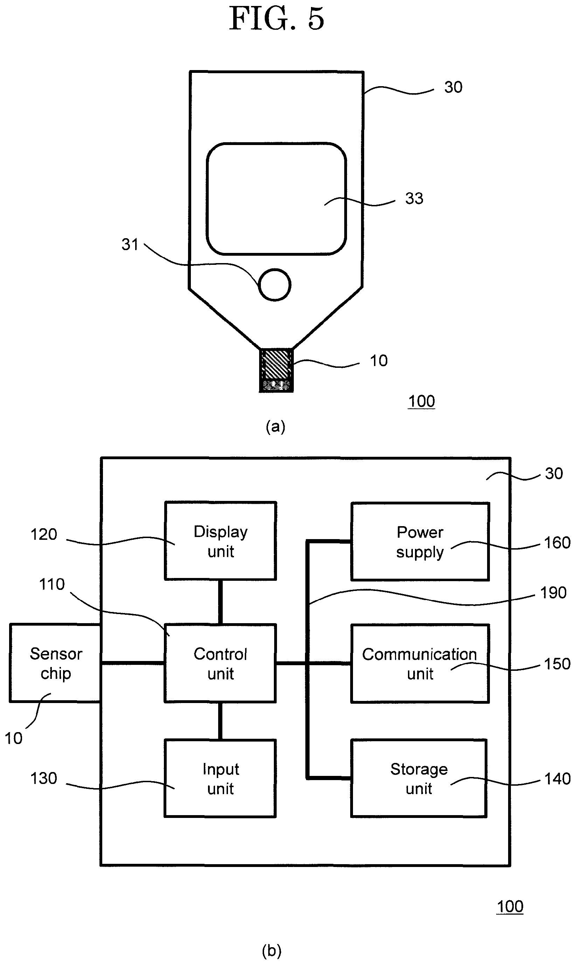

In an embodiment, an EAP measurement sensor using the oxidoreductase of the present invention is provided. FIG. 5(a) is a schematic diagram of a sensor 100 according to an embodiment of the present invention. The sensor is an EAP measurement device using the oxidoreductase of the present invention and includes a sensor chip containing the oxidoreductase, and a measurement unit. A measurement unit 30 may include, for example, a switch 31 serving as an input unit and a display 33 serving as a display unit. The switch 31 may be used, for example, to control ON/OFF of the power supply of the measurement unit 30, or to control the initiation or interruption of the EAP measurement by the sensor 100. For example, the display 33 may display a measured value of EAP, and may include a touch panel as the input unit for controlling the measurement unit 30.

FIG. 5(b) is a block diagram of the sensor 100 according to an embodiment of the present invention. The sensor 100 may include, for example, a control unit 110, a display unit 120, an input unit 130, a storage unit 140, a communication unit 150, and a power supply 160 in the measurement unit 30, which may be electrically connected to each other by a wiring 190. Further, a terminal of the sensor chip 10 to be described later and a terminal of the measurement unit 30 are electrically connected, and the current generated at the sensor chip 10 is detected by the control unit 110. The control unit 110 is a control device which controls the sensor 100 and is composed of, for example, a known central processing unit (CPU) and an operation program which controls the sensor 100. Alternatively, the control unit 110 may include a central processing unit and an operating system (OS) and may include application programs or modules for performing EAP measurements.

The display unit 120 may include, for example, the known display 33, and may display the measured value of EAP, states of the measurement unit 30, and request for operations to a measurer. The input unit 130 is an input device for the measurer to operate the sensor 100, and may be, for example, a touch panel arranged on the switch 31 or the display 33. A plurality of switches 31 may be arranged in the measurement unit 30.

The storage unit 140 is composed of a main storage device (memory) and an auxiliary storage device (hard disk) may be arranged externally. The main storage device (memory) may be composed of a read-only memory (ROM) and/or random access memory (RAM). The operating program, operating system, application program, or module is stored in the storage unit 140 and executed by the central processing unit to configure the control unit 110. The measured values and the current values can be stored in the storage unit 140.

The communication unit 150 is a known communication device that connects the sensor 100 or the measurement unit 30 to external devices (computers, printers, or networks). The communication unit 150 and the external devices are connected by wired or wireless communication. In addition, the power supply 160 is a known power supply device that supplies power to the sensor 100 or the measurement unit 30.

As described above, the quantitation method of EAP according to the present invention, oxidoreductase for quantitation, the quantitation composition, and the quantitation kit can provide a novel quantitation method for quantifying the concentration of EAP, which is a biomarker of depression, a novel enzyme for quantitation, a novel composition for quantitation, a novel kit for quantitation, and a novel sensor for quantitation, by containing the phosphatase and the oxidoreductase.

### (Quantitation Method of EAP using Oxidase Activity)

In the quantitation method of EAP using oxidase activity according to the present embodiment, a phosphatase is made to act on a sample to convert EAP contained in the sample into EA, and an oxidase is made to act on the EA to react a reagent with a produced hydrogen peroxide to determine the concentration of the EAP.

FIG. 1 is referred to. First, a phosphatase is added to the sample and allowed to act on EAP to convert it into EA (S101). An oxidase is added to the sample and allowed to act on the converted EA (S103). The hydrogen peroxide produced by this reaction is detected (S105). EAP is quantified from a detected value of hydrogen peroxide using a previously prepared calibration curve (S107).

Since the step S101 has been described above, a detailed explanation thereof will be omitted. In step S103, the concentration of EAP in the sample is not particularly limited, but may be, for example, 0.01 µM to 10000 µM. An action time may be, for example, 5 seconds to 180 minutes, preferably 0.5 minutes to 60 minutes, more preferably 1 minute to 30 minutes, and still more preferably 1 minute to 10 minutes. A working temperature depends on the optimum temperature of the enzyme to be used, but is, for example, 20°C to 45°C, and the temperature to be used for the ordinary enzyme reaction can be appropriately selected.

Suitable amounts of oxidase used may be added, for example, such that the final concentration is 0.001 U/ml to 500 U/ml, preferably 0.01 U/ml to 100 U/ml. Generally, the lower the concentration of substrate contained in the sample solution, the higher the final concentration of oxidase to be added. A pH at the time of acting the oxidase is preferably adjusted using a buffering agent so as to have a pH suitable for the reaction, considering an optimum pH of oxidoreductase, but is not limited thereto as long as it is an operable pH. The pH at the time of acting the oxidase is, for example, pH3 to pH11, preferably pH5 to pH9. The buffering agents which can be used are similar to those for quantitation of EAP by oxidoreductase.

The present invention provides a method for measuring EAP by measuring a product or consumption by the action of oxidase acting on EA but hydrogen peroxide is exemplified as a preferable measurement object which is a product that is easy to measure. Hydrogen peroxide produced by the action of the oxidase may be detected by a chromogenic substrate or the like, and the examples of the a chromogenic substrate used in the present invention include, for example, ADOS (N-ethyl-N-(2-hydroxy-3-sulfopropyl)-m-anisidine), ALOS (N-ethyl-N-(2-hydroxy-3-sulfopropyl)aniline), TOOS (N-ethyl-N-(2-hydroxy-3-sulfopropyl)-m-toluidine sodium), DA-67 (10-(carboxymethylaminocarbonyl)-3,7-bis(dimethylamino)-phenocyadine), DA-64 (N-(carboxymethylaminocarbonyl)-4,4'-bis(dimethylamino)-diphenylamine), and the like in addition to 4-aminoantipyrine. ADOS, ALOS, and TOOS develop color when condensed with 4-aminoantipyrine. DA-64 and DA-67 do not require 4-aminoantipyrine and develop color when formulated alone. In both cases, the chromogenic reaction is catalyzed by peroxidase. Further, examples of the consumption to be measured include dissolved oxygen, and the amount of dissolved oxygen in the reaction solution can be measured using a dissolved oxygen meter or the like. For example, the degree of color development (absorbance change amount) of the above measurement reagent is measured by a spectrophotometer or a biochemical automatic analyzer or the like, and the EAP contained in the sample can be measured as compared with the absorbance of the standard sample.

In step S105, the amount of EAP in the sample is determined from the detected value of a reaction product using a calibration curve prepared in advance. In this way, EAP in the sample can be quantified.

The sample used in the quantitation method of EAP according to the present invention may be a sample derived from any biological sample, such as blood, plasma, and the like, which may include EAP. The sample may be processed as appropriate. The sample may be concentrated, for example, by a centrifugal concentrator.

However, in such a quantitation method, in the case where the sample containing EAP is also contaminated with a substance other than EAP on which the oxidase for quantitation of EAP can act, there is a possibility that the measured value may cause an error in a positive direction. In particular, the higher the ratio of oxidase acting on such contaminants, the more difficult it becomes to accurately quantify EAP, which is the original substance for quantitation.

In an embodiment, the sample may contain at least one kind of amine other than EAP. Generally, a clinical specimen may contain amines such as EA, β-alanine, taurine, and the like as contaminants. In the quantitation method of EAP according to the present invention, since an oxidase acting on EA is used, when these amines are included in the sample, the oxidase also acts on these amines, making it difficult to accurately quantify EA derived from EAP to be originally measured. Therefore, amines other than EAP contained in the sample are preferably decomposed or eliminated.

In an embodiment, oxidase acting on at least amines is preferably allowed to act on the amine contained in the sample before allowing phosphatase to act on the sample. FIG. 2 is referred to. First, an oxidase is added to the sample, and the amines contained in the sample are decomposed or eliminated (S201). A phosphatase is added to the sample and allowed to act on EAP to convert it into EA (S203). The oxidase added in step S201 acts on the converted EA. The hydrogen peroxide produced by this reaction is detected (S205). EAP is quantified from a detected value of hydrogen peroxide using a previously prepared calibration curve (S207).

In step S201, since the oxidase acting on at least amines is first added to the sample, the amine contained in the sample is decomposed or eliminated by the oxidase. The degree of effect of the error-causing substance in the sample on the measured value in the quantitation method of EAP according to the present invention depends on the amount of free EA contained in the sample. In the case where a sample having a small amount of free EA contained in the sample is measured, the step S201 of eliminating the free EA in advance is not required, and phosphatase can be directly allowed to act on the sample. In this case, it can be inferred that all EA produced in the sample was excised from EAP in the sample by the action of phosphatase.

On the other hand, in the case where a sample containing free EA is measured, it is not possible to distinguish EA liberated by the action of phosphatase from EAP, which is the target material for quantitation, resulting in a measurement error. In order to avoid this measurement error, it is preferred to eliminate free EA in the sample in advance.

Also, in the case of selecting an oxidase having a high specificity for EA and does not substantially act on EAP, EAP is not eliminated in the step S201, which is the target substance for quantitation. However, if it has a possibility to eliminate EAP by the property of the oxidase used, which is the target substance for quantitation in the step S201, erroneous elimination of EAP can be avoided by setting a conditional concentration in which EAP is not eliminated and only EA is eliminated, and performing the step S201.

In step S203, phosphatase is added to the sample and allowed to act on EAP to convert it into EA. Since the step S203 is the same step as the step S101 described above, a detailed explanation thereof will be omitted.

In step S205, the oxidase added in step S201 acts on the EA converted in step S203. The reaction product produced by this reaction can be detected. Since the step S205 is the same as that of the step S103 except that the oxidase already added acts on EA, and is the same as that of the step S105 in that the reaction product in the redox reaction is detected, a detailed explanation thereof will be omitted.

In step S207, EAP is quantified from a detected value of a reaction product using a calibration curve prepared in advance. Since the step S207 is the same as the step S107, a detailed explanation thereof will be omitted.

In the embodiment described above, although an example is shown in which the action of the oxidase added in the amine-elimination step is used as it is, the quantitation method of EAP according to the present invention is not limited to this. An oxidase may be added at the time of the amine-elimination step, and the oxidase may be further added in the EAP quantitation step.

FIG. 3 is referred to. First, a first oxidase is added to the sample, and the amines contained in the sample are decomposed or eliminated (S301). A phosphatase is added to the sample and allowed to act on EAP to convert it into EA (S303). A second oxidase is added to the sample and allowed to act on the converted EA (S305). The hydrogen peroxide produced by this reaction is detected (S307). EAP is quantified from a detected value of hydrogen peroxide using a calibration curve prepared in advance (S309).

The oxidase used in the step S301 for eliminating the free EA in the sample and the oxidase used in the step S305 for quantifying the EA liberated by the action of the phosphatase from EAP, which is the target substance for quantitation, may be different types or the same type. In addition, the oxidoreductase described above may be used to eliminate amines such as the free EA in the sample. Also, the concentration of enzyme suitable for use in elimination, the concentration of enzyme suitable for use in quantitation, or the substrate specificity of enzyme to be used can be appropriately set.

The step S303 may be the same step as the step S101 described above. The step S305 may be the same step as the step S103 described above. The step S307 may be the same step as the step S105 described above. The step S309 may be the same step as the step S107 described above, and a detailed explanation thereof will be omitted.

### <Oxidase>

The oxidase used in the present invention is an oxidizing enzyme that acts on EA as a substrate or an oxidizing enzyme that acts on a substrate structurally similar to EA. Substrates structurally similar to EA include substrates with a CH-NH₂ bond or CH-NH bond, and amine oxidase exists as the enzyme to the substrate.

More specifically, examples of the substrates structurally similar to EA include phenylethylamine, EA, tyramine, benzylamine, histamine, serotonin, spermine, spermidine, β-alanine, γ-aminobutyric acid (GABA), taurine, cadaverine, agmatine, and the like, and examples of the oxidizing enzyme to the substrate include phenylethylamine oxidase (PEAOX), ethanolamine oxidase, tyramine oxidase, benzylamine oxidase, histamine oxidase, serotonin oxidase, spermine oxidase, spermidine oxidase, β-alanine oxidase, γ-aminobutyric acid (GABA) oxidase, taurine oxidase, cadaverine oxidase, agmatine oxidase, and the like, which can be preferably used.

The reaction condition of the oxidase used in the present invention may be any condition as long as it is a condition for acting on EA and efficiently catalyzing an oxidation reaction. However, an enzyme generally has an optimum temperature and optimum pH which exhibit the highest activity. Therefore, the reaction conditions are preferably near the optimum temperature and the optimum pH. For example, a temperature of 37°C and pH 8.5 can be suitably used as the reaction conditions of PEAOX, which will be described later, but is not limited thereto.

As a reaction process of the oxidase used in the quantitation method of EAP according to the present invention, various chemicals may be participated when the oxidase acts on EA. For example, when the oxidase of the present invention acts on EA, oxygen may participate as an electron acceptor for the redox reaction.

The oxidase used in the quantitation method of EAP according to the present invention may be an oxidase produced by a naturally occurring microorganism or oxidase produced by a transformed microorganism. From the viewpoint of efficient mass expression of the enzyme, the enzyme can be efficiently expressed in large quantities by using the transformed microorganism.

For example, the oxidase used in the quantitation method of EAP according to the present invention may be oxidase (AgPEAOX) produced by Arthrobacter globiformis or oxidase produced by E. coli transformed with a plasmid containing a PEAOX gene derived from Arthrobacter globiformis, but by using the E. coli transformed with the plasmid containing the PEAOX gene derived from Arthrobacter globiformis, oxidase can be efficiently expressed in large quantities.

For example, the oxidase used in the quantitation method of EAP according to the present invention may be LcAOX produced by Lichtheimia corymbifera or amine oxidase produced by E. coli transformed with a plasmid containing a LcAOX gene having the base sequence of SEQ ID NO: 3 derived from Lichtheimia corymbifera, but by using the E. coli transformed with the plasmid containing the LcAOX gene derived from Lichtheimia corymbifera, oxidase can be efficiently expressed in large quantities.

Further, for example, the oxidase used in the quantitation method of EAP according to the present invention may be LrHP produced by Lichtheimia ramosa or a hypothetical protein produced by E. coli transformed with a plasmid containing a LrHP gene having the base sequence of SEQ ID NO: 4 derived from Lichtheimia ramosa, but by using the E. coli transformed with the plasmid containing the LrHP gene derived from Lichtheimia ramosa, oxidase can be efficiently expressed in large quantities.

For example, the oxidase used in the quantitation method of EAP according to the present invention may be SrAOX3925 produced by Syncephalastrum racemosum or amine oxidase produced by E. coli transformed with a plasmid containing a SrAOX3925 gene having the base sequence of SEQ ID NO: 5 derived from Syncephalastrum racemosum, but by using the E. coli transformed with the plasmid containing the SrAOX3925 gene derived from Syncephalastrum racemosum, oxidase can be efficiently expressed in large quantities.

For example, the oxidase used in the quantitation method of EAP according to the present invention may be SrAOX3926 produced by Syncephalastrum racemosum or amine oxidase produced by E. coli transformed with a plasmid containing a SrAOX3926 gene having the base sequence of SEQ ID NO: 6 derived from Syncephalastrum racemosum, but by using the E. coli transformed with the plasmid containing the SrAOX3926 gene derived from Syncephalastrum racemosum, oxidase can be efficiently expressed in large quantities.

For example, the oxidase used in the quantitation method of EAP according to the present invention may be SrEAOX produced by Syncephalastrum racemosum or an ethanolamine oxidase produced by E. coli transformed with a plasmid containing a SrEAOX gene having the base sequence of SEQ ID NO: 7 derived from Syncephalastrum racemosum, but by using the E. coli transformed with the plasmid containing the SrEAOX gene derived from Syncephalastrum racemosum, oxidase can be efficiently expressed in large quantities.

In an embodiment, examples of the oxidase used in the quantitation method of EAP according to the present invention include oxidase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to the amino acid sequence (SEQ ID NO: 9) of PEAOX produced by Arthrobacter globiformis, and oxidase having an amino acid sequence in which one or more amino acids are altered or varied, deleted, substituted, added and/or inserted in the amino acid sequence of SEQ ID NO: 9.

In an embodiment, examples of the oxidase used in the quantitation method of EAP according to the present invention include oxidase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to the amino acid sequence (SEQ ID NO: 10) of LcAOX produced by Lichtheimia corymbifera, and oxidase having an amino acid sequence in which one or more amino acids are altered or varied, deleted, substituted, added and/or inserted in the amino acid sequence of SEQ ID NO: 10.

In an embodiment, examples of the oxidase used in the quantitation method of EAP according to the present invention include oxidase which is high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to the amino acid sequence (SEQ ID NO: 11) of LrHP produced by Lichtheimia ramosa, and oxidase having an amino acid sequence in which one or more amino acids are altered or varied, deleted, substituted, added and/or inserted in the amino acid sequence of SEQ ID NO: 11.

In an embodiment, examples of the oxidase used in the quantitation method of EAP according to the present invention include oxidase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to the amino acid sequence (SEQ ID NO: 12) of SrAOX3925 produced by Syncephalastrum racemosum, and oxidase having an amino acid sequence in which one or more amino acids are altered or varied, deleted, substituted, added and/or inserted in the amino acid sequence of SEQ ID NO: 12.

In an embodiment, examples of the oxidase used in the quantitation method of EAP according to the present invention include oxidase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to the amino acid sequence (SEQ ID NO: 13) of SrAOX3926 produced by Syncephalastrum racemosum, and oxidase having an amino acid sequence in which one or more amino acids are altered or varied, deleted, substituted, added and/or inserted in the amino acid sequence of SEQ ID NO: 13.

In an embodiment, examples of the oxidase used in the quantitation method of EAP according to the present invention include oxidase which has high sequence identity (e.g., 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and 99% or more) relative to the amino acid sequence (SEQ ID NO: 14) of SrEAOX produced by Syncephalastrum racemosum, and oxidase having an amino acid sequence in which one or more amino acids are altered or varied, deleted, substituted, added and/or inserted in the amino acid sequence of SEQ ID NO: 14.

Since the amino acid sequence identity of these oxidases is calculated by the same method as the amino acid sequence identity of oxidoreductase, a detailed description thereof will be omitted.

### (Methods for Preparing Oxidase)

Hereinafter, a method for preparing oxidase used in the quantitation method of EAP according to the present invention will be described.

### (Construction of Expression Plasmid)

A plasmid for expressing oxidase used in the quantitation method of EAP according to the present invention is obtained by a commonly used method. For example, DNA is extracted from a microorganism producing oxidase according to the present invention to create a DNA library. From the created DNA library, a DNA fragment encoding the oxidase is identified and isolated. The DNAfragment is amplified by a polymerase chain reaction (PCR) with complementary primers using the isolated DNA fragment as a template to clone a gene encoding the oxidase. The amplified DNA fragment is ligated into a vector to obtain a plasmid having the DNA fragment encoding the oxidase.

Alternatively, the DNA fragment encoding the oxidase is chemically synthesized, and the DNA fragment is ligated to the vector to obtain a plasmid having the DNA fragment encoding the oxidase.

A strain such as E. coli is transformed with the obtained plasmid to obtain a strain such as the E. coli having the DNA encoding the oxidase.

Yeast or a filamentous fungus may be used as a host cell utilized for the expression of the oxidase. The method may be the same as that for the expression of the oxidoreductase described above, and a detailed description thereof will be omitted.

The oxidase may have an amino acid substitution which enhances the reactivity to EAP. For example, it may have an amino acid substitution at a position corresponding to phenylalanine at position 105 and/or a position corresponding to leucine at position 358 of PEAOX derived from Arthrobacter globiformis having an amino acid sequence of SEQ ID NO: 9.

### (Recombinant Expression and Purification of Enzyme)

The expression and purification of the oxidase may be performed by the same method as in the expression and purification of the oxidoreductase described above, and a detailed description thereof will be omitted.

### (Oxidase Activity Measurement)

The method for measuring the activity of an oxidase may be any method as long as it directly or indirectly measures a product by a reaction catalyzed by an oxidase. For example, if a product obtained by the reaction catalyzed by the oxidase and a reagent reacting with the product (hereinafter, a "product reaction reagent") are reacted and a light-absorbing substance generated by the reaction is measured, the enzyme activity can be measured by performing an absorbance measurement.

In an embodiment, for example, an enzyme disclosed in Japanese laid-open patent publication No. 2014-233219 may be used as the oxidase.

### (Composition Containing Oxidase and Kit for Quantification of EAP)

The quantitation method of EAP utilizing the phosphatase and the oxidase according to the present invention may be carried out by providing a composition containing phosphatase, oxidase, and the product reaction reagent, or by combining phosphatase, oxidase, and a commercially available product reaction reagent. For example, the quantitation method may be provided as a quantitation composition of EAP containing phosphatase and oxidase, and as a composition for the quantitation of EAP further including phosphatase and a reagent that reacts with hydrogen peroxide produced by adding oxidase.

In an embodiment, there may be provided a quantitation kit of EAP including phosphatase, an oxidase, and a reagent that reacts with hydrogen peroxide produced by adding oxidase. For example, a quantitation kit of EAP may include a first reagent including phosphatase and an oxidase, and a second reagent including a reagent that reacts with hydrogen peroxide produced by adding oxidase. In addition, the method may further include a third reagent including oxidoreductase or oxidase acting on at least one kind of amine other than ethanolamine phosphate, and the third reagent may be configured to be added to the sample before adding the first reagent and the second reagent.

### (Sensor Chip and Electrode)

In an embodiment, the oxidase of the present invention may be applied, adsorbed, or immobilized on an electrode. Preferably, the oxidase of the present invention is applied, adsorbed or immobilized on a working electrode. In another embodiment, the phosphatase together with oxidoreductase may also be applied, adsorbed, or immobilized on the electrode. Since the configuration of the electrode can be the same as the configuration described for the electrode using the oxidoreductase, a detailed description thereof will be omitted. In addition, the oxidase can be immobilized on the electrode by crosslinking, coating with a dialysis membrane, encapsulation in a polymer matrix, use of a photocrosslinkable polymer, use of an electrically conductive polymer, use of an oxidation/reduction polymer, and the like.

The oxidase of the present invention can be applied to various electrochemical measurement methods by using a potentiostat, a galvanostat, or the like. The electrochemical measurement includes various techniques such as amperometry, potentiometry, and coulometry. For example, by an amperometric method, the concentration of EAP in a sample can be calculated by measuring a current value generated by applying +600 mV to +1000 mV (vs. Ag/AgCI) by a hydrogen peroxide electrode to hydrogen peroxide produced when oxidase reacts with EAP. For example, a calibration curve can be generated by measuring current values for known concentrations of EAP (0 µM, 50 µM, 100 µM, 150 µM, 200 µM) and plotting against concentrations of EAP. The concentration of EAP can be obtained from the calibration curve by measuring the current value of the unknown EAP. For example, a carbon electrode or a platinum electrode can be used as the hydrogen peroxide electrode. The amount of hydrogen peroxide can be quantified by measuring the reduction current value generated by applying - 400 mV to +100 mV (vs. Ag/AgCI) using an electrode immobilized with a reductase such as peroxidase or catalase, instead of the hydrogen peroxide electrode, and the value of EAP can also be measured.

In addition, printed electrodes (sensor chips) can be used to reduce the amount of solution required for the measurement. In this case, the electrodes are preferably formed on a substrate composed of an insulating substrate. The configuration of the sensor chip using oxidase may be the same as the configuration of the sensor chip using oxidoreductase, and a detailed description thereof will be omitted.

### [EAP Measurement Sensor]

In an embodiment, an EAP measurement sensor using the oxidase of the present invention is provided. The sensor is an EAP measurement device using the oxidase of the present invention and includes the sensor chip containing the oxidase, and a measurement unit. The configuration of the EAP measurement sensor using the oxidase may be the same as the configuration of the EAP measurement sensor using the oxidoreductase, and a detailed explanation thereof will be omitted.

As described above, the quantitation method of EAP according to the present invention, oxidase for quantitation, the quantitation composition, and the quantitation kit can provide a novel quantitation method for quantifying the concentration of EAP, which is a biomarker of depression, a novel enzyme for quantitation, a novel composition for quantitation, a novel kit for quantitation, and a novel sensor for quantitation, by containing the oxidase.

### [EXAMPLES]

The following reagents were used.

Reagent: Alkaline phosphatase recombinant, high activity (Roche) 4-aminoantipyrine (4-AA) (Fujifilm Wako Pure Chemical Corporation), taurine (Fujifilm Wako Pure Chemical Corporation), β-alanine (Fujifilm Wako Pure Chemical Corporation), ethanolamine phosphate (EAP) (Tokyo Chemical Industry Co., Ltd.), ethanolamine (EA) (Tokyo Chemical Industry Co., Ltd.), N-ethyl-N-(2-hydroxy-3-sulfopropyl)-3-methylaniline (TOOS) (DOJINDO LABORATORIES), horseradish peroxidase (POD) (TOYOBO Co., Ltd.) and KOD-plus-Neo (TOYOBO Co., Ltd.)

### (Construction of Plasmid for Expression of LrHP)

A plasmid for expression (pKK223-3-LrHP) of hypothetical protein (LrHP, GenBank ID CDS02610.1) having an amino acid sequence of SEQ ID NO: 11 derived from Lichtheimia ramosa was constructed using the In-Fusion (registered trademark) HD Cloning Kit (manufactured by Clontech Laboratories, Inc.).

The fragment of the vector (pKK223-3) was prepared by PCR using pKK223-3-CFP-T7 (refer to the publication of WO2007/125779) as a template and pKK223-3 Hindlll 3Fw(5'-AAGCTTGGCT GTTTTGGCGGATGAGAGAAG-3') and pKK223-3 EcoRI 5Rv(5'-GAATTCTGTT TCCTGTGTGA AATTGTTATC-3') as a primer. After 1.0 µl of Dpnl (manufactured by New England BioLabs, Inc.) was added to the solution after PCR and the solution after PCR was treated for 1 hour at 37°C, followed by agarose gel electrophoresis, and the gel containing the desired fragment (about 4.6 kbp) was sliced and extracted from the gel using illustra (registered trademark) GFX PCR DNA and Gel Band Purification Kit (manufactured by GE Healthcare).

Synthesis of an LrHP gene having a base sequence of SEQ ID NO: 4 was entrusted to Integrated DNA Technologies by dividing into an anterior half portion (Irhp_frag1) in which the DNA sequence of SEQ ID NO: 15 and SEQ ID NO: 16 were sequentially bound in a direction from the 5' end to the 3' end, an intermediate portion (Irhp_frag2) in SEQ ID NO: 17, and the last half portion (Irhp_frag3) in which the DNA sequence of SEQ ID NO: 18 and SEQ ID NO: 19 were sequentially bound in a direction from the 5' end to the 3' end. To take advantage of in-fusion reactions, 15 bases (CATTTAGGGCAAGAT) at the 3' end of Irhp_frag1 and at the 5' end of Irhp_frag2, and 15 bases (CACCATCAACATTTG) at the 3' end of Irhp_frag2 and at the 5' end of Irhp_frag3, respectively, were overlapped.

The vector fragment of pKK223-3 and three LrHP gene fragments were used to perform in-fusion reaction (50°C, 15 minutes) with the composition of Table 1 to obtain the plasmid (pKK223-3-LrHP) for expression of LrHP. The E. coli strain JM109 was transformed with the resulting plasmid.

**[Table 1]**

| | |
|---|---|
| 5 × In-Fusion HD Enzyme Premix | 2.0 µl |
| 40 ng/µl pKK223-3 vector fragment | 1.4 µl |
| 25 ng/µl lrhp_frag1 | 2.2 µl |
| 25 ng/µl lrhp_frag2 | 2.2 µl |
| 25 ng/µl lrhp_frag3 | 2.2 µl |
| Total | 10.0 µl |

### (Recombinant Expression of Enzyme)

An LrHP producing strain was inoculated into 2.5 ml of LB-amp medium (ampicillin concentration 50 µg/ml) charged into a test tube and seed cultured at 37°C and 160 rpm overnight. 1.5 ml of seed culture solution was inoculated into 150 ml of LB-amp medium (ampicillin concentration 50 µg/ml) containing 0.02 mM CuSO₄ and 0.1 mM IPTG charged into a Sakaguchi flask and cultured at 25°C for 20 hours.

A pellet obtained by centrifugation of the 150 ml culture solution at 6,500×g for 10 minutes was resuspended in 20 mM Tris-HCl pH 7.5. After ultrasonic pulverization of the bacterial cell suspension, a supernatant was recovered by centrifugation at 20,400×g for 15 minutes to obtain a crude enzyme solution.

### (Purification of LrHP)

The crude enzyme solution of LrHP was applied to HiScreen (registered trademark) Capto Q (manufactured by GE Healthcare, resin volume 4.7 ml) equilibrated with 20 mM Tris-HCl pH 7.5 to bind to an anion exchange resin.

Thereafter, the resin was washed with 47 ml (10 CV) of 20 mM Tris-HCl (pH 7.5), and 117.5 ml (25 CV) was fed while linearly increasing NaCl concentration contained in 20 mM Tris-HCl (pH 7.5) from 0 mM to 500 mM to elute LrHP bound to the resin.

The eluted fraction was diluted with ion exchanged water three times to reduce salt concentration, and then applied to HiScreen (registered trademark) Capto Q ImpRes (manufactured by GE Healthcare, resin amount 4.7 ml) equilibrated with 20 mM Tris-HCl pH 7.5 to bind to the anion exchange resin.

Thereafter, the resin was washed with 23.5 ml (5 CV) of 20 mM Tris-HCl (pH 7.5), and 141 ml (30 CV) of NaCl concentration contained in 20 mM Tris-HCl (pH 7.5) was fed while linearly increasing from 0 mM to 300 mM to elute LrHP bound to the resin.

The eluted fractions were concentrated by Amicon Ultra Ultracel-30K and purified by HiLoad (registered trademark) 26/60 Superdex 200 columns. 10 mM Bis-Tris-HCl (pH 7.0) with 150 mM NaCl) was used for equilibration of the resins and elution.

The purity of each eluted fraction was assessed by SDS-PAGE and the fraction containing no contaminant protein was recovered to serve as a purified preparation of LrHP.

A reagent for activity measurement of the following composition was prepared.

**[Table 2]**

| | |
|---|---|
| 0.73 mM 4-AA, 7.5 U/ml POD, | 360 µl |
| 150 mM Bicine-NaOH pH 8.0 | |
| H₂O | (200-X) µl |
| 15 mM TOOS | 20 µl |
| Purified LrHP solutions | X µl |
| Total | 580 µl |

### (Verification of EAP to EA Conversion Reaction by ALP)

A sample containing 1 mM EA was mixed with the reaction reagent (pH 8.0) shown in Table 2 in a 1.5 ml microtube at 4°C. 2 µl of ALP (so that the final concentration is 5 U/ml) was added thereto and incubated at 37°C for 5 minutes. 20 µl of LrHP (so that the final concentration is 25 µg/ml) was added thereto, mixed by pipetting, transferred to a cuvette, and immediately set in a spectrophotometer (U-3900, manufactured by Hitachi High-Tech Science Corporation), and the absorbance at 555 nm was measured over time for 300 seconds.

Similarly, a sample containing 1 mM EAP was used, and 2 µl of alkaline phosphatase (ALP) or water was added to the reaction reagent (pH 8.0) (so that the final concentration is 5 U/ml) and incubated at 37°C for 5 minutes. Next, 20 µl of LrHP (so that the final concentration is 25 µg/ml) was added thereto, mixed by pipetting, transferred to a cuvette, and immediately set in a spectrophotometer, and the absorbance at 555 nm was measured over time for 300 seconds.

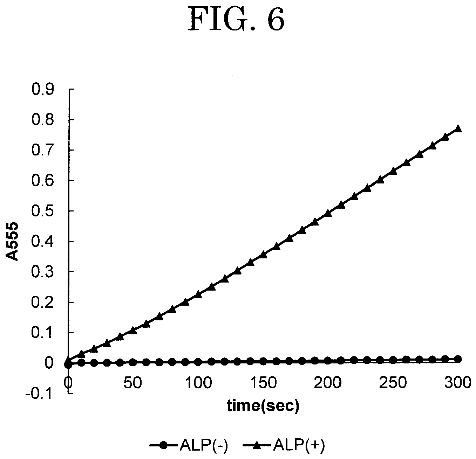

FIG. 6 shows a measurement result of EAP oxidization activity when ALP was added. The time immediately after the addition of LrHP is set to 0 seconds from the start of the measurement. In the sample containing 1 mM EAP, no change in absorbance was observed in the case where ALP was not added (indicated by black circles in the drawing). On the other hand, in the case where ALP was added, an increase in absorbance with time was observed immediately after the addition of LrHP (indicated by black triangles in the drawing). This linear increase in absorbance was similar to the tendency to increase in the case where EA was directly used as a substrate instead of acting phosphatase on EAP. Therefore, it became clear that most of 1 mM EAP was converted to EA by ALP, and the change in absorbance could be measured.

### (Preparation of Plasma Model Solution)

A45 µM EAP solution containing 312 µM EA, 870 µM β-alanine, 1185 µM taurine as the contaminating amine was prepared. Also, EA, β-alanine, and taurine were amines present at relatively high concentrations in plasma, and the concentration of EA was determined by referring to the plasma concentration described in TEST DIRECTORY (https://test-guide-en.srl.info/hachioji_en/test/detail/00080A40H) and the concentration of β-alanine and taurine were determined by referring to the plasma concentration described in MedlinePlus (registered trademark) (https://medlineplus.gov/ency/article/003361.htm) so that the final concentration thereof in the 600 µl measurement system was the same as the plasma concentration.

### (Measurement of EAP combined with ALP and LrHP)

The reaction reagent (pH 8.0) shown in Table 2 was mixed in a 1.5 ml microtube at 4°C and incubated at 37°C for 2 minutes. In this case, 47 µl of LrHP was added (so that the final concentration is 1 mg/ml). 20 µl of 45 µM EAP solution containing EA, β-alanine, taurine was then added thereto, mixed by pipetting, transferred to a cuvette, and immediately set in a spectrophotometer, and the absorbance at 555 nm was measured for 500 seconds. Also, the final concentrations of EA, β-alanine, taurine, and EAP were 10.4 µM, 29 µM, 39.5 µM, and 1.5 µM, respectively. The measurement was paused, 10 µl of alkaline phosphatase (25 U/ml) was added to the cuvette, mixed by pipetting, and immediately set in a spectrophotometer, and the absorbance at 555 nm was measured over time for 500 seconds.

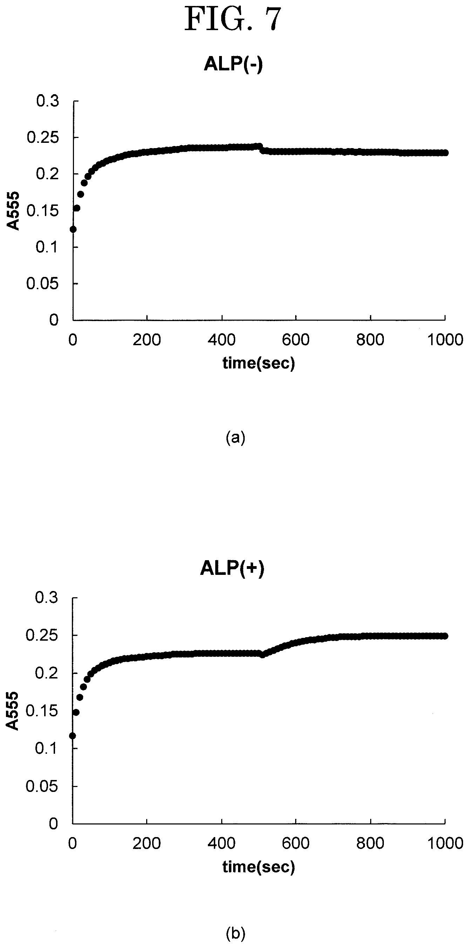

FIG. 7(a) shows a measurement result of a sample obtained by adding 10 µl of water instead of ALP, and FIG. 7(b) shows a measurement result of a sample obtained by adding 10 µl of ALP. When LrHP was added to the sample containing EA and EAP, β-alanine, and taurine, the reaction reached equilibrium after 500 seconds and the EA in the sample was exhausted. When ALP was added after 500 seconds, the EAP in the sample was converted to EA and reacted with LrHP, resulting in a change in absorbance. No change in absorbance occurred without the addition of ALP. Therefore, it became clear that when added in the order of LrHP and ALP, the EA was eliminated by LrHP, and only EAP could be measured (final EAP 1.5 µM). In this case, it was revealed that β-alanine and taurine, which are contaminating amines contained in large amounts in the blood, do not affect the measurement of EAP.

### (Quantification of EAP combined with ALP and LrHP)

The reaction reagent (pH 8.0) shown in Table 2 was mixed in a 1.5 ml microtube at 4°C and incubated at 37°C for 2 minutes. In this case, 47 µl of LrHP was added (so that the final concentration is 1 mg/ml). Four EAP solutions (EAP concentrations of 30 µM EAP, 45 µM, 90 µM, 150 µM) containing β-alanine and taurine were prepared, 580 µl of the reagents (pH 8.0) shown in Table 2 incubated at 37°C for 2 minutes were added to the 20 µl of EAP solutions, mixed by pipetting, transferred to a cuvette, and immediately set in a spectrophotometer, and the absorbance at 555 nm was measured over time for 500 seconds. The measurement was paused, 10 µl of ALP (the final concentration is 25 U/ml) was added to the cuvette, mixed by pipetting, and immediately set in a spectrophotometer, and the absorbance at 555 nm was measured over time for 500 seconds.

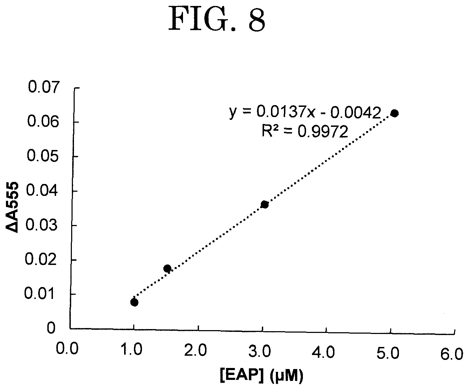

FIG. 8 shows a measurement result of EAP by changing the final concentration of EAP in the sample to 1.0 µM, 1.5 µM, 3.0 µM, and 5.0 µM. Since linearity was observed between the EAP concentration and the absorbance change amount (R² = 0.9972), it was shown that LrHP can measure EAP at final concentrations of 1.0 µM to 5.0 µM in this elimination system.

### (Measurement of EAP combined with ALP and Oxidoreductase Exhibiting Dehydrogenase Activity in EA)

Hereinafter, an example of measuring EAP by combining AgPEAOX exhibiting dehydrogenase activity to EA with ALP will be described.

In addition to the reagent described above, the following reagent was used. Reagents: alkaline phosphatase derived from E.coli C75 (ALP) (TaKaRa), 2,6-dichloroindophenol sodium salt hydrate (DCIP) (Sigma-Aldrich), and 1-Methoxy-5-methylphenazinium methylsulfate (mPMS (DOJINDO LABORATORIES)

### (Construction of Plasmids for Expression of AgPEAOX)

A plasmid (pKK223-3-AgPEAOX) for expression of phenylethylamine oxidase (AgPEAOX, UniProt ID P46881) derived from Arthrobacter globiformis having the amino acid sequence of SEQ ID NO: 9 was prepared using the In-Fusion (registered trademark) HD Cloning Kit (manufactured by Clontech Laboratories, Inc.).

A fragment of the vector (pKK223-3) was prepared by PCR using pKK223-3-CFP-T7 (refer to the publication of WO2007/125779) as a template, pKK223-3 Hindlll 3Fw(5'-AAGCTTGGCT GTTTTGGCGG ATGAGAGAAG-3') and pKK223-3 EcoRI 5Rv(5'-GAATTCTGTT TCCTGTGTGA AATTGTTATC-3') as primers.

1.0 µl of Dpnl (manufactured by New England BioLabs, Inc.) was added to the solution after PCR and the solution was treated for 1 hour at 37°C, followed by agarose gel electrophoresis, and the gel containing the desired fragment (about 4.6 kbp) was sliced and extracted from the gel using illustra (registered trademark) GFX PCR DNA and Gel Band Purification Kit (manufactured by GE Healthcare).

Synthesis of an AgPEAOX gene having a base sequence of SEQ ID NO: 20 was entrusted to Integrated DNA Technologies by dividing into an anterior half portion (agpeaox_1-325) in which the DNA sequence of SEQ ID NO: 15 and SEQ ID NO: 21 were sequentially bound in a direction from 5' end to 3' end, and a last half portion (agpeaox_321-638) in which the DNA sequences of SEQ ID NO: 22 and SEQ ID NO: 19 were sequentially bound in a direction from 5' end to 3' end. 15 bases at the 5' end side of the agpeaox_1-325 (SEQ ID NO: 15: CAGGAAACAGAATTC) and 15 bases at the 3' end side of the agpeaox_321-638 (SEQ ID NO: 19: AAGCTTGGCTGTTTT) indicate sequences derived from the pKK223-3 vector. The 15 bases (ATCACGTACCTGTCC) at the 3' end of agpeaox_1-325 and the 15 bases (ATCACGTACCTGTCC) at the 5' end of agpeaox_321-638 indicate overlapping sequences in the anterior half and the last half of AgPEAOX.

The vector fragment of pKK223-3, and the two AgPEAOX gene fragments were used to perform in-fusion (50°C, 15 min) with the compositions of Table 3 to obtain the plasmid (pKK223-3-AgPEAOX) for expression of AgPEAOX. The E.coli strain JM109 was transformed with the resulting plasmid.

**[Table 3]**

| | |
|---|---|
| 5 × In-Fusion HD Enzyme Premix | 2.0 µl |
| 38.2 ng/µl pKK223-3 vector fragment | 4.6 µl |
| 50 ng/µl agpeaox_1-325 | 1.7 µl |
| 50 ng/µl agpeaox_321-638 | 1.7 µl |
| Total | 10.0 µl |

### (Recombinant Expression of AgPEAOX)

An AgPEAOX producing strain was inoculated into 2.5 ml of LB-amp medium (ampicillin concentration 50 µg/ml) charged into a test tube and seed cultured at 37°C and 160 rpm overnight. 1 ml of seed culture solution was incubated into 150 ml of LB-amp medium (ampicillin concentration 50 µg/ml) containing 0.1 mM CuSO₄ and 0.1 mM IPTG charged into a Sakaguchi flask and cultured at 25°C for 16 hours.

A pellet obtained by centrifugation of the culture solution for 6 Sakaguchi flasks at 6,500×g for 10 minutes was resuspended in 20 mM Tris-HCl pH 8.0 containing 2 mM CuSO₄.

After ultrasonic pulverization of the bacterial cell suspension, a supernatant obtained by centrifugation at 20,400×g for 15 minutes was buffer-replaced with 20 mM Tris-HCl pH 8.0 using Amicon (registered trademark) Ultra Ultracel-30K (manufactured by Millipore) to obtain a crude enzymatic solution of AgPEAOX.

### (Purification of AgPEAOX)

The crude enzyme solution of AgPEAOX was applied to HiScreen (registered trademark) Capto Q (manufactured by GE Healthcare, resin volume 4.7 ml) equilibrated with 20 mM Tris-HCl pH 8.0 to bind to an anion exchange resin.

Thereafter, the resin was washed with 47 ml (10 CV) of 20 mM Tris-HCl (pH 8.0) containing 150 mM NaCl, and 164.5 ml (35 CV) was fed while linearly increasing NaCl concentration contained in 20 mM Tris-HCl (pH 8.0) from 150 mM to 500 mM to elute AgPEAOX bound to the resin.

The eluted fractions were concentrated by Amicon Ultra Ultracel-30K and purified by HiLoad (registered trademark) 26/60 Superdex 200 columns. 20 mM Tris-HCl (pH 8.0) was used for equilibration of the resin and elution.

The purity of the eluted fraction was assessed by SDS-PAGE, and the fraction containing no contaminant protein was recovered and used as a purified preparation of AgPEAOX.

A reagent for activity measurement of the following composition was prepared.

**[Table 4]**

| | |
|---|---|
| 250 mM Bicine-NaOH pH 8.5 | 480 µl |
| H₂O | (518.8-X) µl |
| 1.75 mM DCIP | 137.2 µl |
| 5 mM mPMS | 24 µl |
| Purified AgPEAOX solutions | X µl |
| Total | 1160 µl |

### (Quantification of EAP combined with ALP and AgPEAOX)

The reaction reagent (pH 8.5) shown in Table 4 was mixed in a 1.5 ml microtube at 4°C and incubated at 37°C for 2 minutes. In this case, 28.2 µl of AgPEAOX was added (so that the final concentration is 1 mg/ml). Three EAP solutions (EAP concentrations of 6 mM, 15 mM, 30 mM) containing β-alanine and taurine were prepared, 1160 µl of the reaction reagents (pH 8.5) shown in Table 3 incubated at 37°C for 2 minutes were added to the 40 µl of EAP solutions, mixed by pipetting, transferred to a cuvette, and immediately set in a spectrophotometer, and the absorbance at 520 nm was measured over time for 500 seconds. The measurement was paused, 7.48 µl of ALP (the final concentration is 3.12 U/ml) was added to the cuvette, mixed by pipetting, and immediately set in a spectrophotometer, and the absorbance at 520 nm was measured over time for 500 seconds.

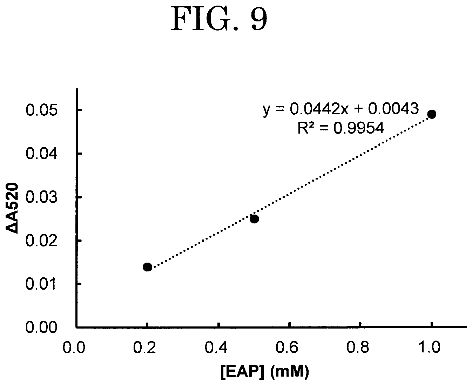

FIG. 9 shows a measurement result of EAP by changing the final concentration of EAP in the sample to 0.2 mM, 0.5 mM, and 1.0 mM. Since linearity was observed between the EAP concentration and the absorbance change amount (R² = 0.9954), it was shown that AgPEAOX can measure EAP at final concentrations of 0.2 mM to 1.0 mM in this elimination system.

### REFERENCE SIGNS LIST

1: working electrode, 3: counter electrode, 5: reference electrode, 7: wiring portion, 9: terminal, 10: sensor chip, 11: substrate, 13: spacer, 15: cover, 19: reaction layer, 30: measurement unit, 31: switch, 33: display, 100: sensor, 110: control unit, 120: display unit, 130: input unit, 140: storage unit, 150: communication unit, 160: power supply, 190: wiring

## Claims

1. A quantitation method of ethanolamine phosphate comprising:
allowing phosphatase to act on a sample to convert ethanolamine phosphate contained in the sample into ethanolamine; and
allowing oxidoreductase to act on the ethanolamine.

2. The quantitation method of ethanolamine phosphate according to claim 1,
wherein the sample includes at least one kind of amine other than the ethanolamine phosphate,
the oxidoreductase is an oxidoreductase acting on at least the amine, and
the oxidoreductase is allowed to act on the at least one kind of amine before allowing phosphatase to act.

3. The quantitation method of ethanolamine phosphate according to claim 1,
wherein a mediator is reduced by allowing the oxidoreductase to act on the ethanolamine, and
a concentration of the ethanolamine phosphate is determined by quantifying the reduced mediator.

4. The quantitation method of ethanolamine phosphate according to claim 1,
wherein the oxidoreductase is an oxidase, and
a concentration of the ethanolamine phosphate is determined by allowing the oxidoreductase to act on the ethanolamine and quantifying generated hydrogen peroxide or consumed oxygen.

5. The quantitation method of ethanolamine phosphate according to claim 2,
wherein a first oxidoreductase allowed to act on the ethanolamine, and a second oxidoreductase allowed to act on the at least one kind of amine are contained.

6. An oxidoreductase, wherein the oxidoreductase is selected from oxidoreductases belonging to EC NO: 1.4 or EC NO: 1.5, and
the oxidoreductase is used in the quantitation method of ethanolamine phosphate according to any one of claims 1 to 5.

7. A quantitation composition for ethanolamine phosphate comprising:
a phosphatase; and
an oxidoreductase acting on the ethanolamine.

8. A quantitation kit for ethanolamine phosphate comprising:
a phosphatase; and
an oxidoreductase acting on the ethanolamine.

9. A sensor chip comprising:
a working electrode; a counter electrode; a reference electrode; and
a reaction layer arranged on the working electrode, the counter electrode, and the reference electrode,
wherein the reaction layer includes an oxidoreductase acting on the ethanolamine, and
the sensor chip is used in the quantitation method of ethanolamine phosphate according to any one of claims 1 to 5.

10. A sensor comprising: the sensor chip according to claim 9.
